(19) Europäisches Patentamt
European Patent Office
Office européen des brevets

(11) EP 1 718 658 B1

(12) **EUROPÄISCHE PATENTSCHRIFT**

(45) Veröffentlichungstag und Bekanntmachung des Hinweises auf die Patenterteilung:
**28.04.2010 Patentblatt 2010/17**

(21) Anmeldenummer: **04804319.4**

(22) Anmeldetag: **27.12.2004**

(51) Int Cl.:
***C07H 17/07*** *(2006.01)* ***A61K 8/60*** *(2006.01)*

(86) Internationale Anmeldenummer:
**PCT/EP2004/014729**

(87) Internationale Veröffentlichungsnummer:
**WO 2005/068484 (28.07.2005 Gazette 2005/30)**

(54) **FLAVONOID-KOMPLEXE MIT CYCLODEXTRINEN**

FLAVONOID COMPLEXES COMPRISING CYCLODEXTRINS

COMPLEXES FLAVONOIDES COMPORTANT DES CYCLODEXTRINES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **19.01.2004 DE 102004002787**

(43) Veröffentlichungstag der Anmeldung:
**08.11.2006 Patentblatt 2006/45**

(73) Patentinhaber: **Merck Patent GmbH**
**64293 Darmstadt (DE)**

(72) Erfinder:
- **WIRTH, Corinna**
  **64283 Darmstadt (DE)**
- **ROSSKOPF, Ralf**
  **64839 Muenster (DE)**
- **BUCHHOLZ, Herwig**
  **60599 Frankfurt (DE)**

(56) Entgegenhaltungen:
**WO-A-02/069926**

- **BUSCHMANN H-J ET AL: "APPLICATIONS OF CYCLODEXTRINS IN COSMETIC PRODUCTS: A REVIEW" JOURNAL OF COSMETIC SCIENCE, SOCIETY OF COSMETIC CHEMISTS, NEW YORK, NY,, US, Bd. 53, Nr. 3, Mai 2002 (2002-05), Seiten 185-191, XP008013888 ISSN: 1525-7886**
- **LOFTSSON T ET AL: "CYLODEXTRINS IN TOPICAL DRUG FORMULATONS: THEORY AND PRACTICE" INTERNATIONAL JOURNAL OF PHARMACEUTICS, AMSTERDAM, NL, Bd. 225, Nr. 1/2, August 2001 (2001-08), Seiten 15-30, XP001023910 ISSN: 0378-5173**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 718 658 B1

## Beschreibung

Die Erfindung betrifft Komplexe bestimmter Flavonoid-Derivate, Zubereitungen, die solche Derivate enthalten, entsprechende Verfahren zur Herstellung der Flavonoid-Derivate bzw. der diese enthaltenden Zubereitungen und deren Verwendung, insbesondere zur Pflege, Konservierung oder Verbesserung des allgemeinen Zustandes der Haut oder Haare.

In der Internationalen Patentanmeldung WO 02/69926 werden Verbindungen

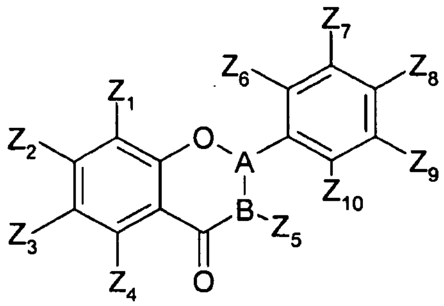

worin

$Z_1$ bis $Z_4$ und

$Z_6$ bis $Z_{10}$ jeweils unabhängig voneinander H, OH, $CH_3COO$, Alkoxy, Hydroxyalkoxy, Mono- oder Oligoglykosidreste bedeuten und

wobei die Alkoxy- und Hydroxyalkoxygruppen verzweigt und unverzweigt sein und 1 bis 18 C-Atome aufweisen können,

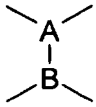 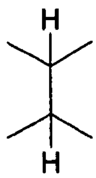 oder 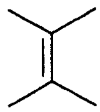

bedeutet,

$Z_5$ ein Mono- oder Oligoglykosidrest ist, wobei an diesen Glykosidrest jeweils über eine Gruppe -O- mindestens ein Rest gebunden ist, der ausgewählt ist aus

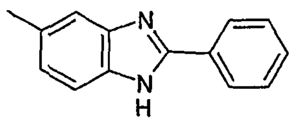 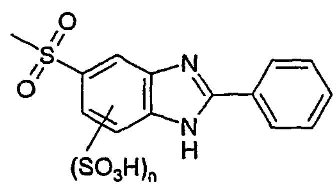

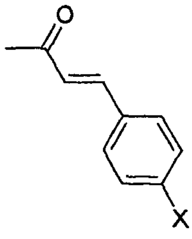 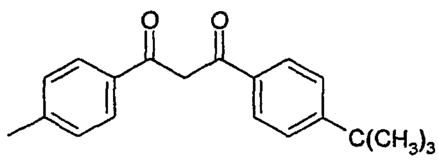

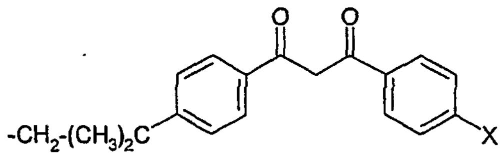

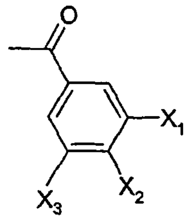

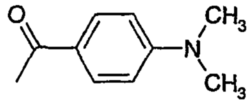

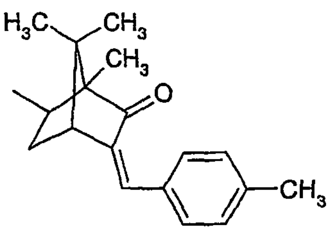

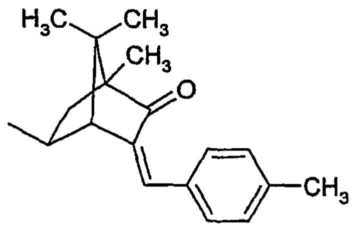

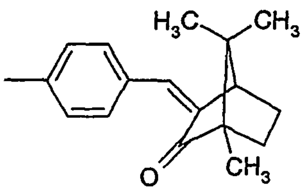

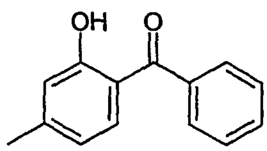

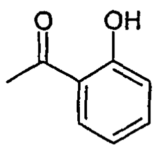

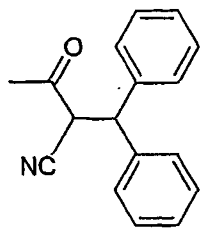

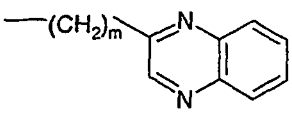

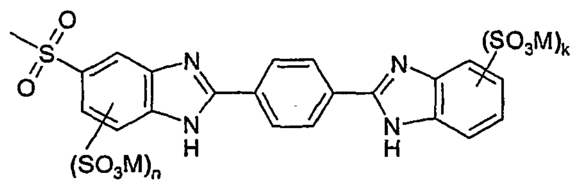

worin X, $X_1$, $X_2$ und $X_3$ jeweils unabhängig voneinander OH,
$CH_3COO$, einen Alkoxyrest mit 1 bis 8 C-Atomen oder einen Monoglykosidrest bedeuten, n 0, 1, 2 oder 3, m 0 oder 1, k 0, 1, 2, 3 oder 4 und M H, Na oder K ist, und
worin ein oder mehrere Wasserstoffatome in den OH-Gruppen der in den Substituenten $Z_1$ bis $Z_{10}$ genannten Glykosidreste jeweils unabhängig voneinander auch durch Acetyl oder Alkylreste ersetzt sein können und wobei an ein oder mehrere Hydroxygruppen der in den Substituenten $Z_1$ bis $Z_{10}$ genannten Reste jeweils unabhängig voneinander auch Sulfat oder Phosphat gebunden sein kann, beschrieben. Diese Verbindungen eignen sich zum Einsatz in kosmetischen und pharmazeutischen Zubereitungen. Insbesondere wird in der WO 02/69926 die Eignung dieser Verbindungen zur Verwendung als UV-Filter, sowie die Verwendung als Wirkstoff zum Schutz gegen oxidativen Stress und zur Vermeidung der Hautalterung beschrieben. Weiter wird beschrieben, dass diese Verbindungen anti-allergische, anti-inflammatorische, entzündungshemmende und anti-irritative Eigenschaften zeigen und somit zur Behandlung oder vorbeugenden Behandlung von Allergien, Entzündungen und Irritationen, insbesondere der Haut, verwendet werden können. Besonders bevorzugte Vertreter sind dabei Kaempferol-3-(6"-galloylglucosid) sowie Kaempferol-3-(6"-p-coumarylglucosid), welches auch als Tilirosid bezeichnet wird.

In der DE 195 44 905 A1 wird z.B. ein Verfahren zur Herstellung von Pflanzenextrakten enthaltend Tilirosid beschrieben sowie die Verwendung der Pflanzenextrakte in Arzneimitteln und Lebensmittelprodukten.

In der DE 199 22 287 A1 wird Tilirosid als ein Ausgangsflavonoid zur Herstellung von Tilirosidestern, deren Säureeinheit 3 bis 30 C-Atome enthält, beschrieben. Diese Ester werden in Kosmetika verwendet. In der DE 199 22 287 A1 werden jedoch keine Zubereitungen enthaltend Tilirosid beschrieben.

In der älteren Europäischen Patentanmeldung mit dem Anmelde-Aktenzeichen EP 03015616.0 wird die Eignung dieser Verbindungen zur Behandlung von Ekzemen beschrieben. Dabei sind die Verbindungen besonders vorteilhaft in der Behandlung atopischer Ekzeme, wie insbesondere Milchschorf, Neurodermitis, Prurigo und Dermatitis sicca. Die Verbindungen können dabei die akuten Symptome stark vermindern, die Häufigkeit des Auftretens von akuten Symptomen reduzieren und allgemein zur Verbesserung des Hautbildes beitragen.

Bei der Anwendung dieser Verbindungen besteht der Wunsch nach Darreichungsformen, die sich leichter in Zubereitungen einarbeiten lassen, deren Zubereitungen eine erhöhte Lagerstabilität zeigen, bzw. in denen die Bioverfügbarkeit der Verbindungen erhöht ist.

Jetzt wurde überraschend gefunden, dass die Komplexierung dieser Verbindungen mit Cyclodextrinen zu Produkten führt, welche die genannten Anforderungen in hervorragender Weise erfüllen.

Ein erster Gegenstand der vorliegenden Erfindung sind daher Komplex Verbindungen nach Formel I

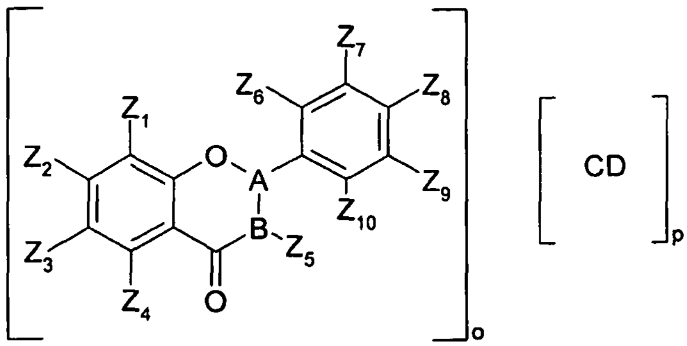

I

wobei es sich bei dem Flavonoid-Anteil

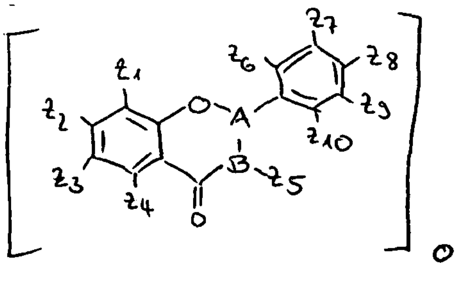

um eine Verbindung des Formel IIA1 oder IIA2 handelt

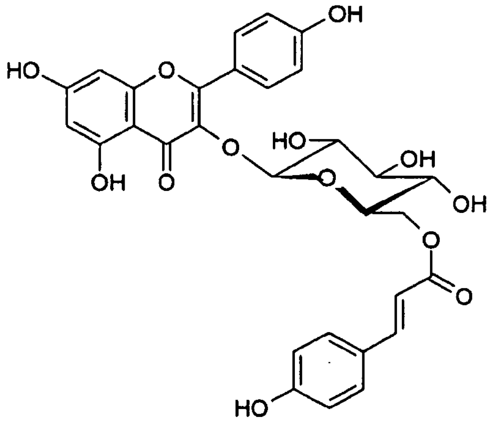

IIA1

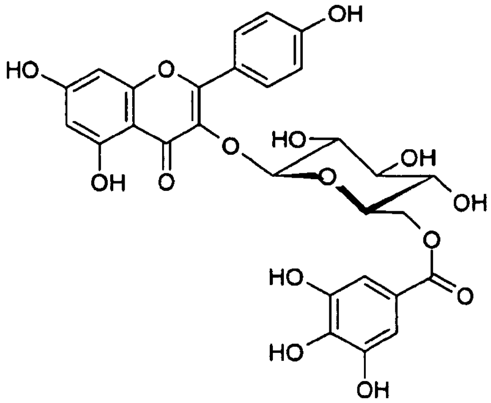

IIA2

CD steht für ein gegebenenfalls an ein oder mehreren Hydroxygruppen $C_{1-24}$-Alkyl- oder $C_{1-24}$-Hydroxyalkyl-substituiertes gammaCyclodextrin
o steht für die Zahl 1 und
p steht für eine Zahl aus dem Bereich 1,75 bis 2,1,

Ein zweiter Gegenstand der vorliegenden Anmeldung sind Zubereitungen enthaltend einen geeigneten Träger, **dadurch gekennzeichnet, dass** die Zubereitungen

- 0,005 bis 99 Gew.-% einer Komplex-Verbindung nach Formel I gemäß Anspruch 1 enthält oder die Zubereitung
- 0,002 bis 70 Gew.-% Cyclodextrin und
- 0,001 bis 60 Gew.-% mindestens einer Verbindung der Formel IIA1 oder IIA2 oder ihrer topisch verträglicher Salze und/oder Derivate enthält.

Bei den erfindungsgemäßen Zubereitungen handelt es sich dabei üblicherweise entweder um topisch anwendbare Zubereitungen, beispielsweise kosmetische oder dermatologische Formulierungen, oder um Arzneimittel bzw. Nahrungsmittel bzw. Nahrungsergänzungsmittel. Die Zubereitungen enthalten einen kosmetisch oder dermatologisch oder pharmazeutisch oder Nahrungsmittel-geeigneten Träger und je nach gewünschtem Eigenschaftsprofil optional weitere geeignete Inhaltsstoffe.

Cyclodextrine sind aus 6, 7, 8 oder noch mehr $\alpha$-1,4-verknüpften Glucoseeinheiten aufgebaut, wobei die Cyclohexaamylose (alpha- oder $\alpha$-Cyclodextrin) sich durch die Struktur

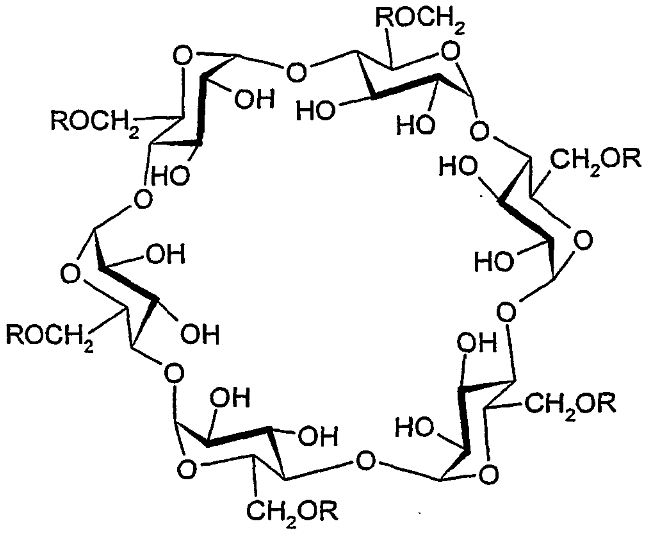

auszeichnet. Die Cycloheptaamylose (beta- oder β-Cyclodextrin) zeichnet sich durch die Struktur

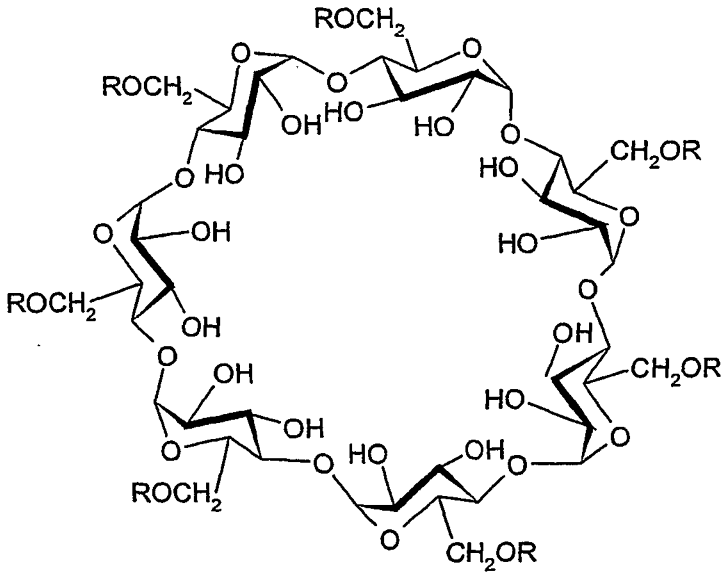

aus. Die Cyclooctaamylose (gamma- oder γ-Cyclodextrin) zeichnet sich durch die Struktur

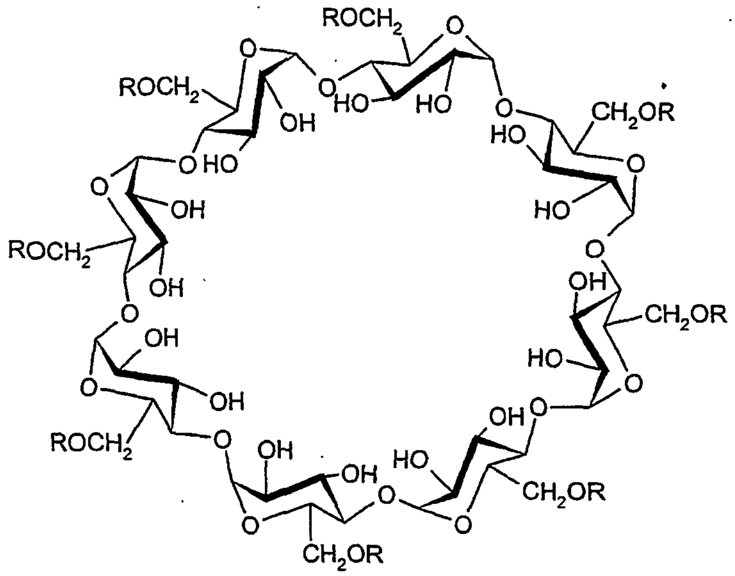

aus. Die Cycloenneaamylose (delta- oder δ-Cyclodextrin) zeichnet sich durch die Struktur

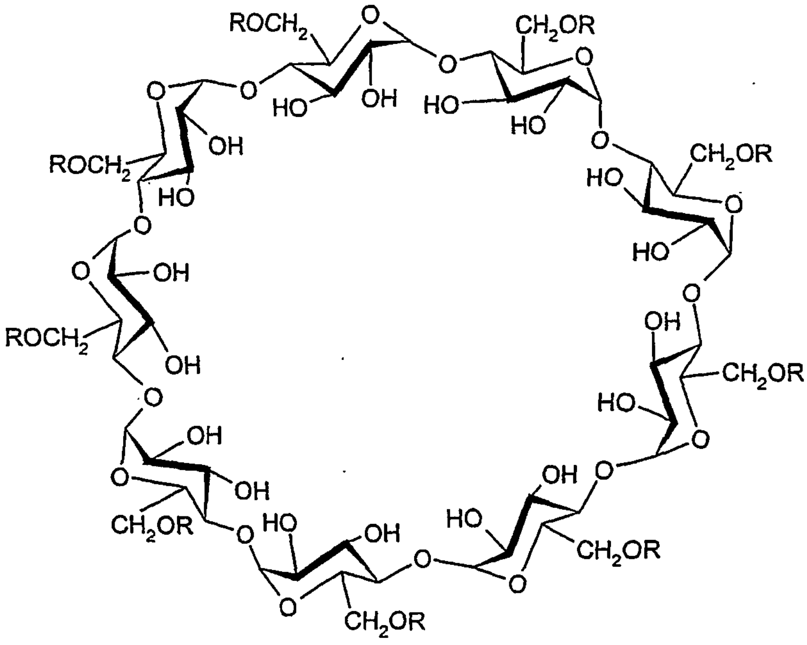

aus. Cyclodextrine können in der underivatisierten Form (R = H) oder auch in derivatisierter Form, z. B. in der Position R alkoxyliert, Hydroxyalkyliert oder alkyliert, insbesondere propoxyliert oder methyliert vorliegen.

Bioflavonoid-Cyclodextrin-Komplexe sind dabei im Prinzip bekannt:

- K. Miyake, H. Arima et al. (Pharm. Dev. Techn. 5(3) 2000, 399-407 beschreiben 1:1 - Komplexe von Rutin mit beta-Cyclodextrinen und deren Löslichkeits- bzw. Freisetzungsverhalten. Dabei zeigte sich, dass der beta-Cyclodextrin-Komplex und der 2-Hydroxypropyl-beta-cyclodextrin-Komplex besonders stabil sind. Alpha- und gamma-Cyclodextrin-Komplexe sind nach dieser Publikation generell schlechter zur Komplexierung von Rutin geeignet als beta-cyclodextrin-Komplexe.

- T. K. Nguyen, H. Galons, C. Chemtob, Congr. Int. Technol. Pharm., 6th (1992), Vol 5, 408-16408-416 beschreiben ebenfalls verschiedene Cyclodextrin-Komplexe von Rutin. Als besonders gut löslich erweist sich hier der 2,6-Di-O-methyl-beta-cyclodextrin-Komplex., wobei Komplexe mit Molverhältnissen Rutin:Cyclodextrin von 1:1 und 1:2 beschrieben werden.
- Komplexe von Isoflavonen in Sojabohnen bzw. fermentierten Sojabohnen mit beta- und gamma-Cyclodextrinen werden in der europäischen Patentanmeldung EP-A- 796 624 beschrieben. Die Komplexbildung erhöht die Löslichkeit der Isoflavone und reduziert deren Bitterkeit.
- Rutin- Komplexe mit beta- und gamma-Cyclodextrinen und ihre Verwendung als Antioxidans werden in der Japanischen Patentanmeldung JP 05/9137499 beschrieben.
- Getränke enthaltend Cyclodextrin-Komplexe von Quercetin-Glycosiden werden in der Japanischen Patentanmeldung JP 07/075536 beschrieben.
- In der Japanischen Patentanmeldung JP 05/186344 werden Zusammensetzungen enthaltend Vitamin C und Cyclodextrin-Komplexe von Vitamin P, welche die Bioabsorption von Vitamin C verbessern, beschrieben. Es werden Komplexe von Rutin, Hesperidin und Eriocitrin, wie beispielsweise ein Rutin-beta-Cyclodextrin-Komplex mit Molverhältnis 1.2 beschrieben.
- Die Wirkung eines Komplexes von 3-Methoxyquercetin mit Hydroxypropyl-beta-cyclodextrin auf nasale Epithelzellen wird in S. Dimova, R. Mugabowindekwe et al. Int. J. Pharm. 26381-2) 2003, 95-103 untersucht.
- Beta-Cyclodextrin-Komplexe verschiedener Flavonoide (Hesperetin, Hesperidin, Naringenin, Naringin) werden in R. Ficarra, S. Tommasini et al.; J. Pharm. Biomed. Analysis 29(6) 2002, 1005-1014 charakterisiert.
- In R.-L. Wang, Yu Yang et al.; Yingyong Huaxue 19(7) 2002 702-704 wird die Stabilität und Löslichkeit verschiedener Beta-Cyclodextrin-Komplexe verschiedener Flavonoide (Rutin, Quercetin, Morin) verglichen. Methyl-beta-cyclodextrin-Komplexe erwiesen sich dabei als besonders gut löslich.
- K. Hostettmann, M. Lederer und A. Marston; Phytochemical Analysis 1186) 2000, 380-382 untersuchen die eluierende Wirkung von 2-Hydroxypropyl-beta-cyclodextrin auf Flavonoide, die auf Cellulose absorbiert sind.

Es hat sich für den Fachmann nicht vorhersehbar herausgestellt, dass Zubereitungen zur topischen Anwendung enthaltend die oben genannten Komplex-Verbindungen der Formel I bzw. Verbindungen der Formel IIA1 oder IIA2 und gegebenenfalls an ein oder mehreren Hydroxygruppen $C_1$-$_{24}$-Alkyl- oder $C_{1-24}$-Hydroxyalkyl-substituierte gamma-Cyclodextrine, wie insbesondere Hydroxypropyl-γ-Cyclodextrin, oder Gemische aus Cyclodextrinen, welche mindestens zu 30 Gew.%, bezogen auf das Gesamtgewicht des Cyclodextringemisches an den o.g. γ-Cyclodextrinen enthalten, den Nachteilen des Standes der Technik abhelfen.

Weiterhin vorteilhaft ist es, wenn der Gehalt an Cyclodextrinen 0,01-20,0 Gew.%, bevorzugt 0,05-10,0 Gew. %, besonders bevorzugt 0,1-5,0 Gew.%, jeweils bezogen auf das Gesamtgewicht der Zusammensetzung beträgt. Der Anteil der Verbindungen der Formel IIA1 oder IIA2, in der Zubereitung beträgt dabei vorzugsweise von 0,01 bis 20 Gew. %, besonders bevorzugt von 0,05 bis 10 Gew.% und insbesondere bevorzugt von 0,1 bis 5 Gew.% bezogen auf die gesamte Zubereitung. Ganz außerordentlich bevorzugt beträgt der Anteil der Verbindungen der Formel IIA1 odes IIA2 in der Zubereitung von 0,1 bis 2 Gew.% bezogen auf die gesamte Zubereitung.

Die Wirkstoffkombinationen gemäß der Erfindung bzw. kosmetische oder dermatologische Zubereitungen, solche Wirkstoffkombinationen enthaltend, sind in jeglicher Hinsicht überaus befriedigende Präparate. Es war für den Fachmann nicht vorauszusehen, dass die Zubereitungen gemäß der Erfindung

- Verbindungen der Formel I in erhöhter Bioverfügbarkeit bereitstellen,
- besser die Barriereeigenschaften der Haut erhalten oder wiederherstellen,
- besser der Hautaustrocknung entgegenwirken und
- die Haut besser vor Umwelteinflüssen schützen als die Zubereitungen des Standes der Technik.

Bei Anwendung der erfindungsgemäß verwendeten Komplex-Verbindungen bzw. kosmetischer oder topischer dermatologischer Zubereitungen mit einem wirksamen Gehalt an erfindungsgemäß verwendeten Wirkstoffkombinationen ist in überraschender Weise eine wirksame Behandlung, aber auch eine Propylaxe

- von defizitären, sensitiven oder hypoaktiven Hautzuständen oder defizitären, sensitiven oder hypoaktiven Zustände von Hautanhangsgebilden,
- von umweltbedingten (Rauch, Smog, reaktive Sauerstoffspecies, freie Radikale) und insbesondere lichtbedingten negativen Veränderungen der Haut und der Hautanhangsgebilde,
- von lichtbedingten Hautschäden,
- von Juckreiz,
- von trockenen Hautzuständen und Hornschichtbarrierestörungen,
- von entzündlichen Hautzuständen sowie atopischem Ekzem, seborrhoischem Ekzem, polymorpher Lichtdermatose,

Psioriasis, Vitiligo
möglich. Die erfindungsgemäßen Komplex-Verbindungen bzw. kosmetische oder topische, dermatologische Zubereitungen mit einem wirksamen Gehalt an erfindungsgemäßen Komplex-Verbindungen dienen aber auch in überraschender Weise

- zur Beruhigung von empfindlicher oder gereizter Haut,
- zur Stimulation der intrazellulären DNA-Synthese, insbesondere bei defizitären oder hypoaktiven Hautzuständen,
- zur Steigerung der hauteigenen Schutz- und Reparaturmechanismen (beispielsweise für dysfunktionelle Enzyme, DNA, Lipide, Proteine),
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenrationsprozesse in der verletzten Haut zu fördern.

Erfindungsgemäß ist daher auch die Verwendung der Komplex-Verbindungen nach Formel I bzw. der Zubereitungen enthaltend die Verbindungen gemäß Formel IIA1 oder IIA2 und gegebenenfalls an ein oder mehreren Hydroxygruppen $C_{1-24}$-Alkyl- oder $C_{1-24}$-Hydroxyalkyl-substituierte gamma-cyclodextrine

- zur kosmetischen oder dermatologischen Behandlung oder Prophylaxe unerwünschter Hautzustände,
- zur Prophylaxe und Behandlung von entzündlichen Hautzuständen - auch dem atopischen Ekzem,
- zum Hautschutz bei empfindlich determinierter trockener Haut,
- zum Schutz der Haut vor Photoreaktionen,
- zur Behandlung und Prophylaxe von sensiblen Hautzuständen,
- zur Stärkung des desoxidativen Hauteigenschutzes,
- und/oder zur Verbesserung des Schutzes der Haut vor Umwelteinflüssen.

Die Komplex-Verbindungen bzw. Zubereitungen enthaltend die Wirkstoffkombination gemäß der Erfindung wirken in all diesen Verwendungen synergistisch in Bezug auf die einzelnen Komponenten.

Erfindungsgemäß vorteilhaft ist die Verwendung von gegebenenfalls an ein oder mehreren Hydroxygruppen $C_{1-24}$-Alkyl- oder $C_{1-24}$-Hydroxyalkyl-substituierten gamma - Cyclodextrinen zur Erhöhung der Löslichkeit von Verbindungen der Formel IIA1 und IIA2. Weiterhin vorteilhaft ist die Verwendung von gegebenenfalls an ein oder mehreren Hydroxygruppen $C_{1-24}$-Alkyl- oder $C_{1-24}$-Hydroxyalkyl-substituierten gamma - Cyclodextrinen zur Verbesserung der biologischen Wirksamkeit von Verbindungen der Formel IIA1 und IIA2.

Einige Flavonoid-Anteile der Verbindungen der Formel I bzw. Verbindungen der Formel IIA1 und IIA2 wie z.B. Tilirosid können aus Pflanzen gewonnen werden, z.B. aus den Pflanzen der Gattung Althaea, Aristolochia, Helianthemum, Lindera, Magnolia, Platanus, Potentilla, Quercus, Rosa, Sida, Sorbus und/oder Tilia. Diese Verbindungen können entweder in isolierter Form oder auch in nicht isolierter Form weiterverarbeitet werden, also z.B. in Form eines Extrakts oder in Form eines aufgereinigten Extrakts oder auch in Form der aus dem Pflanzenextrakt hergestellten Reinsubstanz in Zubereitungen eingearbeitet werden. Unter den genannten Gattungen sind folgende Arten bevorzugt: Althaea officinalis, Althaea rosea, Aristolochia heterophylla, Helianthemum glomeratum, Lindera megaphylla, Magnolia salicifolia, Platanus acerifolia, Platanus occidentalis, Potentilla anserina, Quercus pubescens, Quercus suber, Quercus laurifolia, Quercus ilex, Quercus imbricaria, Quercus virginiana, Rosa pomifera, Sida rhombifolia, Sida poeppigiana, Sida cordifolia, Sida glaziovii, Sorbus pendula, Tilia argenta und Tilia cordata.

Wenn die erfindungsgemäße Zubereitung Tilirosid enthält, ist diese Verbindung in einer weiteren bevorzugten Ausführungsform in der Form eines Pflanzenextrakts, eines aufgereinigten Pflanzenextrakts oder in Form der aus dem Pflanzenextrakt hergestellten Reinsubstanz zur Herstellung der Zubereitung verwendet worden.
In derartigen Zubereitungen enthält der Pflanzenextrakt beispielsweise 1 bis 100 Gew.% Tilirosid. In einer Ausführungsform enthält der Pflanzenextrakt vorzugsweise 5 bis 90 Gew.% Tilirosid. In einer weiteren Ausführungsform enthält der Pflanzenextrakt vorzugsweise 30 bis 100 Gew.%, besonders bevorzugt 60 bis 100 Gew.% und insbesondere bevorzugt 90 bis 100 Gew.% Tilirosid.
In einer weiteren bevorzugten Ausführungsform ist der Pflanzenextrakt durch Extraktion der Pflanze Sida glaziovii gewonnen worden.

Bei allen erfindungsgemäßen Verwendungen, in denen Tilirosid zum Einsatz kommt, kann Tilirosid z.B. in Form einer synthetisch gewonnenen Substanz, in Form eines Pflanzenextrakts, eines aufgereinigten Pflanzenextrakts oder als Einzelsubstanz bzw. in Form einer aus dem Pflanzenextrakt gewonnenen Reinsubstanz verwendet werden. In einer bevorzugten Ausführungsform wird Tilirosid hierbei in der Form eines Pflanzenextrakts, eines aufgereinigten Pflanzenextrakts oder in Form der aus dem Pflanzenextrakt hergestellten Reinsubstanz verwendet.

Die Flavonoid-Anteile der Verbindungen der Formel I bzw. Verbindungen der Formel IIA1 und IIA2 können nach Methoden, die dem Fachmann wohl bekannt und in der Literatur beschrieben sind (z.B. in Standard-Werken wie Houben-Weyl, Methoden der organischen Chemie, Georg-Thieme-Verlag, Stuttgart), gewonnen oder hergestellt werden.

Beispielsweise kommt Tilirosid in Pflanzen vor und kann durch Extraktion gewonnen werden. Die Herstellung der Pflanzenextrakte erfolgt durch übliche Methoden der Extraktion der Pflanzen bzw. Pflanzenteile. Geeignete Extraktionsverfahren können sein: Mazeration, Remazeration, Digestion, Bewegungsmazeration, Wirbelextraktion, Ultraschallextraktion, Gegenstromextraktion, Perkolation, Reperkolation, Evakolation, Diakolation oder Festflüssig-Extraktion unter kontinuierlichem Rückfluss, die in einem Soxhlet-Extraktor durchgeführt wird.

Als Lösungsmittel für die Extraktion kann beispielsweise Wasser oder ein Alkohol verwendet werden.

Es ist dem allgemeinen Wissen des Fachmanns zuzurechnen, wie diese Extraktionen im Einzelnen durchgeführt werden und die erhaltenen Rohextrakte durch allgemein geläufige Methoden aufgereinigt werden können.

Ein möglicher Syntheseweg für Tilirosid ist z.B. auch in B. Vermes, H. Wagner, Stud. Org. Chem. (Amsterdam) (1982), Volume date 1981, 11 (Flavonoids, Bioflavonoids), 161-167 und in B. Vermes, V.M. Chari, H.Wagner, Helv. Chim. Acta (1981), 64(4), 1964-1967 beschrieben.

Die Synthese von Tilirosid ist in Schema 1 wiedergegeben. 4',7-Dibenzylkaempferol **(1)** [H. Wagner, H. Danninger, O. Seligmann, M. Nógrádi, L. Farkas, N. Farnsworth, Chem. Ber. 103 (1978) 3768] wird in Gegenwart von $Ag_2CO_3$ und Pyridin mit 2,3,4-Tri-O-acetyl-6-O-chloroacetyl-β-D-glucopyranosylbromid **(2)** zu Verbindung **3** umgesetzt. Die Verbindung **2** kann nach der in D.Y. Gagniere, P.J.A. Wottero, Carbohydrate Res. 28 (1973) 1965 beschriebenen Methode hergestellt werden. Die katalytische Debenzylierung und anschließende vorsichtige Acetylierung der Verbindung **3** liefert Verbindung **4** aus der nach Entfernung der Chloracetyl-Gruppe mit Thioharnstoff Verbindung **5** erhalten werden kann. In dieser Verbindung ist lediglich eine Hydroxylgruppe frei, sodaß die Veresterung der Verbindung **5** selektiv verlaufen kann. Die Veresterung mit dem Säurechlorid p-Acetylcoumaroylchlorid **6** kann in einer Mischung aus Pyridin und Dichloromethan durchgeführt werden. Damit die Veresterung vollständig abläuft ist ein Überschuß an Säurechlorid und eine lange Reaktionszeit (ca. 96h) bei Raumtemperatur notwendig. Der letzte Schritt, die selektive Verseifung der 7 Acetylgruppen in Verbindung **7,** kann nach der in G. Zemplén, Chem. Ber. 59 (1926) 1258 beschriebenen Methode durchgeführt werden. Hierbei wird unter Verwendung einer katalytischen Menge an $NaOCH_3$ und einer kalkulierten Menge an Methanol gearbeitet.

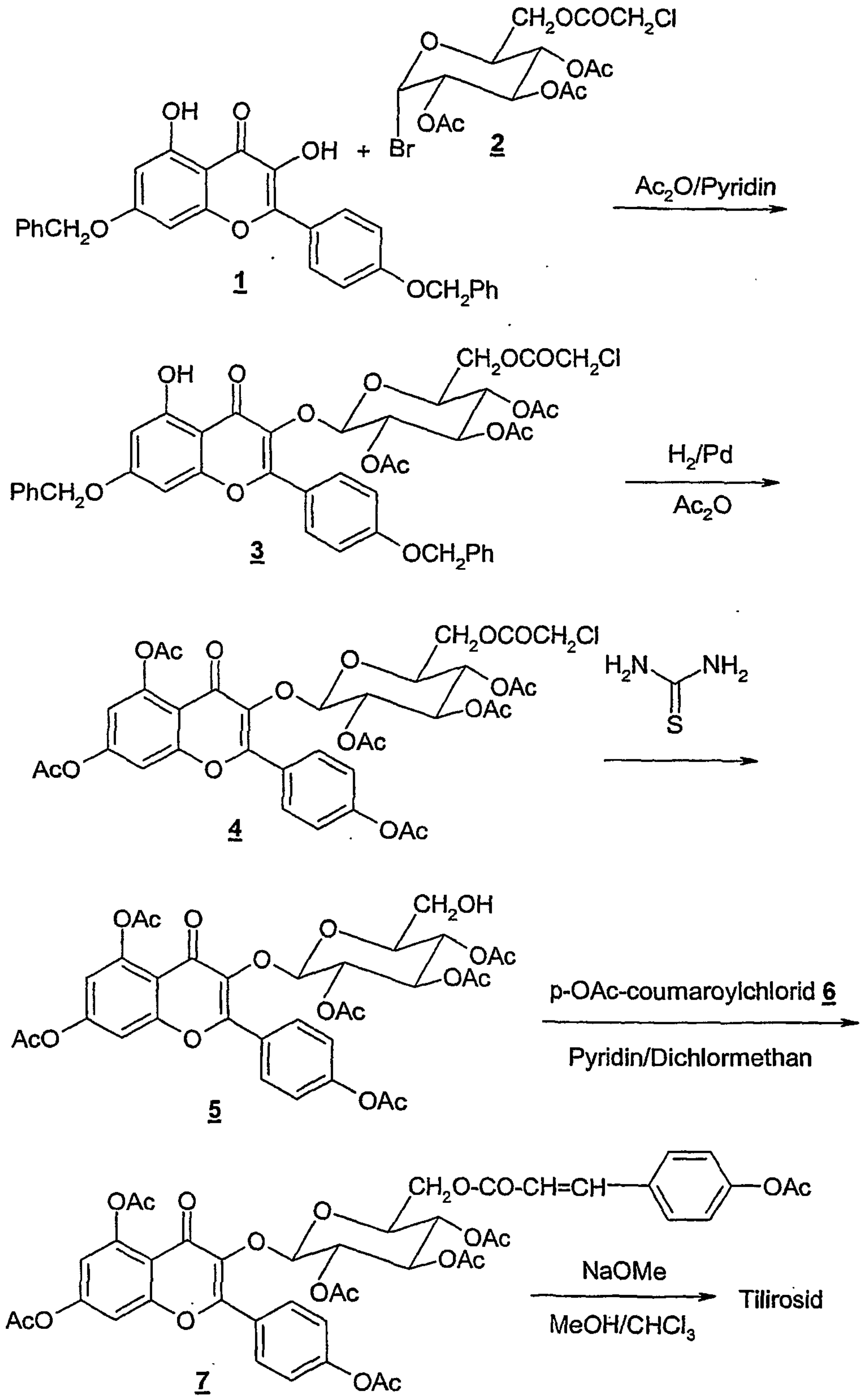


**Schema 1**

Ph = Phenyl, Ac = $CH_3CO$, Me = Methyl

Andere Flavonoid-Anteile der Verbindungen der Formel I bzw. Verbindungen der Formel IIA1 und IIA2 können

durch routinemäßige Abwandlung der in Schema 1 gezeigten Synthese erhalten werden. Hierbei werden je nach Zielmolekül andere Edukte, d.h. andere gegebenenfalls geschützte Flavonoide, Zuckerkomponenten und Reste, die an die Zuckerkomponente angehängt werden sollen, verwendet.

Die Veresterung glykosidischer OH-Gruppen mit aromatischen Sulfonsäure-Einheiten kann beispielsweise nach der in A.B. Foster et al., J. Chem. Soc. (1954) 3625-3629 beschriebenen Methode erfolgen. Hiernach kann die Zuckerkomponente z.B. mit einem entsprechenden aromatischen Sulfonsäurechlorid in Pyridin zur Reaktion gebracht werden.

Die Veretherung glykosidischer OH-Gruppen mit aromatischen Resten kann beispielsweise nach der in P. Beraud et al., Tetrahedron Let. 30(3) (1989) 325-326 beschriebenen Methode erfolgen. Bei dieser Mitsunobu-Reaktion findet die Veretherung beispielsweise derart statt, dass die Zuckerkomponente zusammen mit Triphenylphosphin $PPh_3$ in Pyridin gelöst und mit einer entsprechenden Phenolkomponente und Diethylazodicarboxylat zur Reaktion gebracht wird.

Die Veretherung glykosidischer OH-Gruppen mit Resten gesättigter Kohlenwasserstoffe kann beispielsweise nach der in M. Goebel et al., Tetrahedron 53(9) (1997) 3123-3134 beschriebenen Methode erfolgen. Die Veretherung findet z.B. derart statt, dass die Zuckerkomponente in trockenem Dimethylformamid unter Inertgas vorsichtig mit Natriumhydrid versetzt und danach mit einem geeigneten Alkylierungsreagenz wie z.B. einem entsprechenden Bromid vorsichtig umgesetzt wird.

Die Komplex-Verbindungen der Formel I lassen sich herstellen, indem Verbindungen der Formel IIA1 und IIA2 mit Cyclodextrinen in Lösung, vorzugsweise bei erhöhter Temperatur, umgesetzt werden. Ein entsprechendes Verfahren ist ein weiterer Gegenstand der vorliegenden Erfindung.

Es hat sich gezeigt, dass Komplexe mit etwa 2 mol Cyclodextrin pro mol Flavonoid der Formel IIA1 odes IIA2 die erfindungsgemäßen Anforderungen in besonderer Weise erfüllen. Daher ist es erfindungsgemäß bevorzugt, wenn in Formel I o gleich 1 ist und p in dem Bereich 1,75 bis 2,1, vorzugsweise wenn p gleich 2 ist.

Entsprechende Verbindungen können hergestellt werden, wenn das Cyclodextrin im Überschuß oder genau im Molverhältnis 2 : 1 bezogen auf das Flavonoid eingesetzt wird.

In einer bevorzugten Ausführungsform der vorliegenden Erfindung handelt es sich bei der Zubereitung um eine Zubereitung zum Schutz von Körperzellen gegen oxidativen Stress, insbesondere zur Verringerung der Hautalterung, **dadurch gekennzeichnet, dass** sie neben den ein oder mehreren Verbindungen nach Formel I oder der Formel IIA1 oder IIA2 ein oder mehrere weitere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynek® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit Verbindungen der Formel I oder Formel II in solchen Zusammensetzungen überlicherweise in Verhältnissen im Bereich von 1000:1 bis 1: 1000, bevorzugt in Mengen von 100:1 bis 1:100 eingesetzt.

Die erfindungsgemäßen Zubereitungen können als weitere Inhaltsstoffe Vitamine enthalten. Bevorzugt sind

Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin. Vitamine werden dabei mit Verbindungen der Formel I oder Formel IIA1 oder IIA2 üblicherweise in Verhältnissen im Bereich von 1000:1 bis 1:1000, bevorzugt in Mengen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind weiter Verbindungen der Formel III

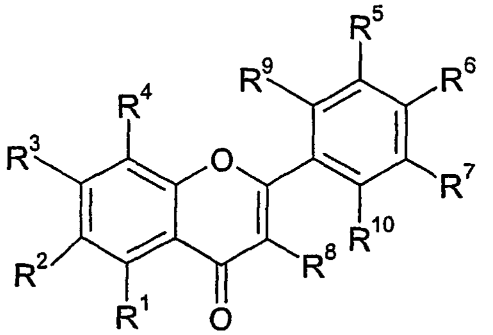

III

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen,

wobei die Hydroxygruppe an ein primäres oder sekundäres Kohlenstoffatom der Kette gebunden sein kann und weiter die Alkylkette auch durch Sauerstoff unterbrochen sein kann, und/oder

- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für

  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an ein primäre

oder sekundäre Kohlenstoffatome der Kette gebunden sein können und weiter die Alkylkette auch durch Sauerstoff unterbrochen sein kann, und/oder
- $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
- Mono- und/oder Oligoglycosylreste,

mit der Maßgabe, dass mindestens 4 Reste aus $R^1$ bis $R^7$ stehen für OH und dass im Molekül mindestens 2 Paare benachbarter Gruppen -OH vorliegen,
- oder $R^2$, $R^5$ und $R^6$ für OH und die Reste $R^1$, $R^3$, $R^4$ und $R^{7-10}$ für H stehen,

wie sie in der älteren Deutschen Patentanmeldung DE 10244282.7 beschrieben sind.

Erfindungsgemäß insbesondere bevorzugte Zubereitungen enthalten neben den Verbindungen der Formel I bzw. Formel IIA1 odes IIA2 auch UV-Fliter.

Bei Einsatz der als UV-A-Filter insbesondere bevorzugten Dibenzoylmethanderivate in Kombination mit den Verbindungen der Formel I bzw. Formel IIA1 oder IIA2 ergibt sich ein zusätzlicher Vorteil: Die UVempfindlichen Dibenzoylmethanderivate werden durch die Anwesenheit der Verbindungen der Formel I bzw. Formel IIA1 odes IIA2 zusätzlich stabilisiert. Ein weiterer Gegenstand der vorliegenden Erfindung ist daher die Verwendung der Verbindungen gemäß Formel I bzw. Formel IIA1 oder IIA2 zur Stabilisierung von Dibenzoylmethanderivaten in Zubereitungen.

Prinzipiell kommen alle UV-Filter für eine Kombination mit den erfindungsgemäßen Verbindungen der Formel I bzw. Formel IIA1 oder IIA2 in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 %, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- $\alpha$-(Trimethylsilyl)-$\omega$-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)

- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Weitere geeignete UV-Filter sind auch Methoxyflavone ensprechend der älteren Deutschen Patentanmeldung DE 10232595.2.

Organische UV-Filter werden in der Regel in einer Menge von 0,5 bis 20 Gewichtsprozent, vorzugsweise 1 - 15 %, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex®T-AQUA, Eusolex® T-AVO), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-methoxy-benzophenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.

Durch Kombination von einer oder mehrerer Verbindungen der Formel I bzw. Formel II mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Dies können Chromon-Derivate sein. Dabei werden vorzugsweise bestimmte Chromen-2-on-Derivate, die sich als Wirkstoffe zur vorbeugenden Behandlung von menschlicher Haut und menschlicher Haare gegen Alterungsprozesse und schädigende Umwelteinflüssen eignen, unter der Bezeichnung Chromon-Derivate verstanden. Sie zeigen gleichzeitig ein niedriges Irritationspotential für die Haut, beeinflussen die Wasserbindung in der Haut positiv, erhalten oder erhöhen die Elastizität der Haut und fördern somit eine Glättung der Haut. Diese Verbindungen entsprechen vorzugsweise der Formel IV

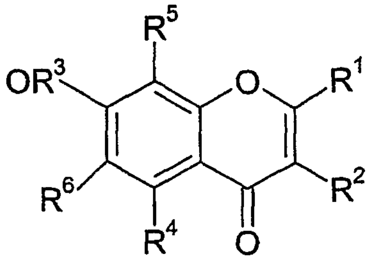

IV

wobei
$R^1$ und $R^2$ gleich oder verschieden sein können und ausgewählt sind aus

- H, -C(=O)-$R^7$, -C(=O)-O$R^7$,
- geradkettigen oder verzweigten $C_1$- bis $C_{20}$-Alkylgruppen,
- geradkettigen oder verzweigten $C_3$- bis $C_{20}$-Alkenylgruppen, geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/ oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können, $R^3$ steht für H oder geradkettige oder verzweigte $C_1$- bis $C_{20}$-Alkylgruppen, $R^4$ steht für H oder O$R^8$,
  $R^5$ und $R^6$ gleich oder verschieden sein können und ausgewählt sind aus
- H, -OH,
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen,

wobei die Hydroxygruppe an ein primäres oder sekundäres Kohlenstoffatom der Kette gebunden sein kann und weiter die Alkylkette auch durch Sauerstoff unterbrochen sein kann und
$R^7$ steht für H, geradkettige oder verzweigte $C_1$- bis $C_{20}$-Alkylgruppen, eine Polyhydroxy-Verbindung, wie vorzugsweise einen Ascorbinsäurerest oder glycosidische Reste und
$R^8$ steht für H oder geradkettige oder verzweigte $C_1$- bis $C_{20}$-Alkylgruppen,
wobei mindestens 2 der Substituenten $R^1$, $R^2$, $R^4$-$R^6$ verschieden von H sind oder mindestens ein Substituent aus $R^1$ und $R^2$ für -C(=O)-$R^7$ oder - C(=O)-O$R^7$ steht.

Der Anteil an einer oder mehreren Verbindungen ausgewählt Cromon-Derivaten in der erfindungsgemässen Zubereitung beträgt vorzugsweise von 0,001 bis 5 Gew.%, besonders bevorzugt von 0,01 bis 2 Gew.% bezogen auf die gesamte Zubereitung.

Weiter kann es bevorzugt sein, wenn die erfindungsgemäße Zubereitung mindestens ein Repellent enthält, wobei das Repellent vorzugsweise ausgewählt aus N,N-Diethyl-3-methylbenzamid, 3-(Acetyl-butyl-amino)-propionsäure Ethylester, Dimethylphthalat, Butopyronoxyl, 2,3,4,5-bis-(2-Butylen)-tetrahydro-2-furaldehyd, N,N-Caprylsäurediethylamid, N,N-Diethylbenzamid, o-Chlor-N,N-diethylbenzamid, Dimethylcarbat, Di-n-propylisocinchomeronat, 2-Ethylhexan-1,3-diol, N-Octyl-bicyclohepetendiecarboximid, Piperonyl-butoxid, 1-(2-Methylpropyloxycarbonyl)-2-(hydroxyethyl)-piperidin oder Mischungen davon, wobei es insbesondere bevorzugt ausgewählt ist aus N,N-Diethyl-3-methylbenzamid, 3-(Acetyl-butyl-amino)-propionsäure-ethylester 1-(2-Methylpropyloxycarbonyl)-2-(hydroxyethyl)-piperidin oder Mischungen davon.

Bei den erfindungsgemäßen Zubereitungen, die Repellentien enthalten, handelt es sich dabei vorzugsweise

um Insektenabwehrmittel. Insektenabwehrmittel werden in Form von Lösungen, Gelen, Stiften, Rollern, Pump-Sprays und Aerosol-Sprays angeboten, wobei Lösungen und Sprays den Hauptteil der im Handel erhältlichen Produkte bilden. Basis für diese beiden Produktformen sind meist alkoholische bzw. wässrig-alkoholische Lösungen unter Zusatz fettender Substanzen und leichter Parfümierung.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (9985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel V eingesetzt,

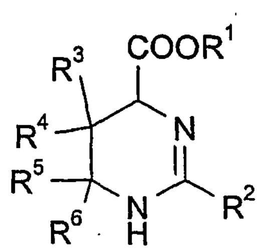

V

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den Verbindungen der Formel I eingesetzt, wobei Verhältnisse im Bereich 1:10 bis 10:1 besonders bevorzugt sind.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel I zusätzlich eine Aryloxim, vorzugsweise 2-Hydroxy-5-methyllaurophenonoxim enthalten, zeigen überraschende anti-inflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

In einer weiteren ebenfalls bevorzugten Ausführungsform der vorliegenden Erfindung enthält die erfindungsgemäße Zubereitung mindestens einen Selbstbräuner.

Als vorteilhafte Selbstbräuner können unter anderem eingesetzt werden:

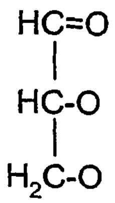

Glycerolaldehyd

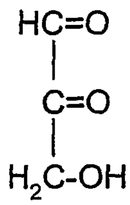

Hydroxymethylglyoxal

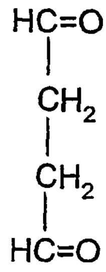

γ-Dialdehyd

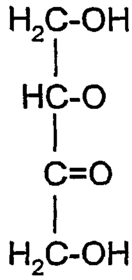

Erythrulose

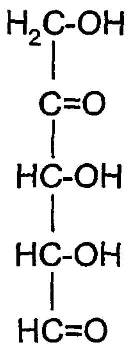

6-Aldo-D-Fructose

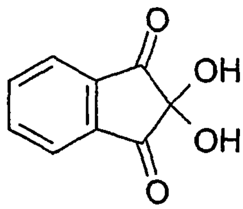

Ninhydrin

Ferner ist das 5-Hydroxy-1,4-naphtochinon (Juglon) zu nennen, das aus den Schalen frischer Walnüsse extrahiert wird

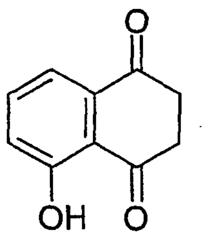

5-Hydroxy-1,4-naphtochinon (Juglon)

sowie das in den Henna-Blättern vorkommende 2-Hydroxy-1,4-naphtochinon (Lawson).

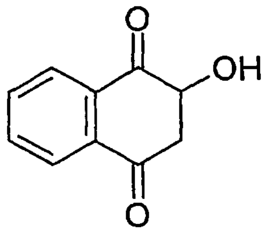

2-Hydroxy-1,4-naphtochinon (Lawson)

Ganz besonders bevorzugt ist das 1,3-Dihydroxyaceton (DHA), ein im menschlichen Körper vorkommender dreiwertiger Zucker und dessen Derivate.

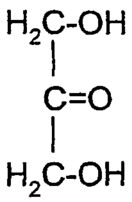

1,3-Dihydroxyaceton (DHA)

Ferner können die erfindungsgemäßen Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramerinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | grün |
| Acid Green 1 | 10020 | Grün |
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol Solvent Red 3 | 11920 | Orange |
| | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |

(fortgesetzt)

| Chemische oder sonstige Bezeichnung | CIN | Farbe |
|---|---|---|
| Pigment Brown 1 | 12480 | Braun |
| 4-(2'-Methoxy-5'sulfonsäurediethylamid-1'-phenylazo)-3-hydroxy-5"-chloro-2",4"-dimethoxy2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4-[4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-β-Apo-8'-Carotinaldehyd ($C_{30}$) | 40820 | Orange |

(fortgesetzt)

| Chemische oder sonstige Bezeichnung | CIN | Farbe |
|---|---|---|
| trans-Apo-8'-Carotinsäure ($C_{30}$)-ethylester | 40850 | Orange |
| Canthaxanthin | 40850 | Orange |
| Acid Blue 1 | 42045 | Blau |
| 2,4-Disulfo-5-hydroxy-4'-4''-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violet |
| Basic Violet 2 | 42520 | Violet |
| 2'-Methyl-4'-(N-ethyl-N-m-sulfobenzyl)-amino-4''-(N-diethyl)-amino-2-methyl-N-ethylIN-m-sulfobenzyl-fuchsonimmonium | 42735 | Blau |
| 4'-(N-Dimethyl)-amino-4''-(N-phenyl)-aminonaphtho-N-dimethylfuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |
| 3-(2'-Methylphenylamino)-6-(2'-methyl-4'-sulfophenylamino)-9-(2''-carboxyphenyl)-xantheniumsalz | 45190 | Violet |
| Acid Red 50 | 45220 | Rot |
| Phenyl-2-oxyfluoron-2-carbonsäure | 45350 | gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | SchwarZ |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | orange |
| Indigo | 73000 | Blau |
| Indigo-disulfosäure | 73015 | Blau |
| 4,4'-Dimethyl-6,6'-dichlorthioindigo | 73360 | Rot |

(fortgesetzt)

| Chemische oder sonstige Bezeichnung | CIN | Farbe |
|---|---|---|
| 5,5'Dichlor-7,7'-dimethylthioindigo | 73385 | violett |
| Quinacridone Violet 19 | 73900 | violett |
| Pigment Red 122 | 73915 | Rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | Rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |
| Mangananimoniumdiphosphat | 77742 | violett |
| Manganphosphat; $Mn_3(PO_4)_2 \cdot 7\ H_2O$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |

(fortgesetzt)

| Chemische oder sonstige Bezeichnung | CIN | Farbe |
|---|---|---|
| Benzopyryliumsalzem, Anthocyane | | rot |
| Aluminium-, Zink-, Magnesium- und Calciumstearat | | weiß |
| Bromthymolblau | | blau |

Es kann ferner günstig sein, als Farbstoff eine oder mehrerer Substanzen aus der folgenden Gruppe zu wählen: 2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, β-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

    1. "Fischsilber" (Guanin/Hypoxanthin-Mischkristalle aus Fischschuppen) und
    2. "Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung/Schichtdicke | Farbe |
|---|---|---|
| **Silberweiße Perlglanzpigmente** | $TiO_2$: 40-60 nm | silber |
| **Interferenzpigmente** | $TiO_2$: 60-80 nm | gelb |
| | $TiO_2$: 80-100 nm | rot |
| | $TiO_2$: 100-140 nm | blau |
| | $TiO_2$: 120-160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente , die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihre Partikelgröße von 40-80 $\mu$m zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.% bis 30-Gew.%, vorzugsweise von 0,5 bis 15 Gew.%, insbesondere von 1,0 bis 10 Gew.% gewählt, jeweils bezogen auf das Gesamtgewicht der Zubereitungen.

Alle Verbindungen oder Komponenten, die in den Zubereitungen verwendet werden können, sind entweder bekannt und käuflich erwerbbar oder können nach bekannten Verfahren synthetisiert werden.

Die eine oder die mehreren Verbindungen der Formel I können in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fett-Ikoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemisch der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

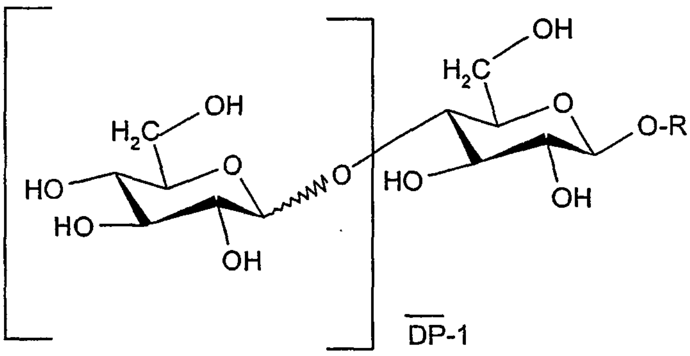

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen,

welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

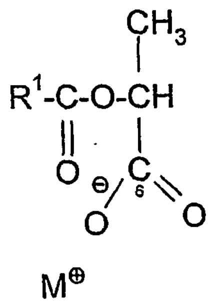

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

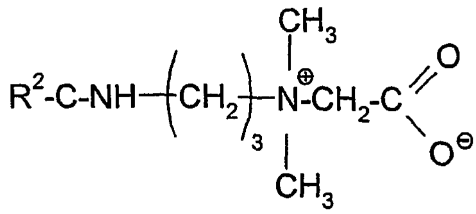

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft **dadurch gekennzeichnet, dass** das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise aufdie Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18) stearyiether (Steareth-18), Polyethylenglycol(19)stearylether (Steareth-19), Polyethylenglycol(20)-stearylether (Steareth-20), Polyethylenglycol(1 2)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethyfenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol (19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol (15)oleylether (Oleth-15), Polyethylenglycol(12)laurylether (Laureth-12), Polyethyfenglycol(12)-isolaurylether (Isolaureth-12), Polyethylenglycol(1 3)cetylstearylether (Ceteareth-13), Polyethylenglycol(14)cetylstearylether (Ceteareth-14), Polyethylenglycol(1 5)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethyfenglycol(20)stearat, Polyethylenglycol(21)stearat,

Polyethylenglycol(22)stearat, Polyethylenglycol(23)stearat,

Polyethylenglycol(24)stearat, Polyethylenglycol(25)stearat,

Polyethylenglycol(12)isostearat, Polyethylenglycol(13)isostearat,

Polyethylenglycol(14)isostearat, Polyethylenglycol(15)isostearat,

Polyethylenglycol(16)isostearat, Polyethylenglycol(17)isostearat,

Polyethylenglycol(18)isostearat, Polyethylenglycol(19)isostearat,

Polyethylenglycol(20)isostearat, Polyethylenglycol(21)isostearat,

Polyethylenglycol(22)isostearat, Polyethylenglycol(23)isostearat,

Polyethylenglycol(24)isostearat, Polyethylenglycol(25)isostearat,

Polyethylenglycol(12)oleat, Polyethylenglycol(13)oleat,

Polyethylenglycol(14)oleat, Polyethylenglycol(15)oleat,

Polyethylenglycol(16)oleat, Polyethylenglycol(17)oleat,

Polyethylenglycol(1 8)oleat, Polyethylenglycol(19)oleat,

Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25) Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethyfenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -Milch vorliegt und außer der oder den Verbindungen der Formel I bzw. Formel II A1 oder IIA2 beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren,

Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann außer der oder den Verbindungen der Formel I bzw. Formel IIA1 oder IIA2 verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Weitere Gegenstände der vorliegenden Erfindung sind ein Verfahren zur Herstellung einer Zubereitung, welches dadurch gekennzeichnet ist, dass mindestens eine Verbindung der Formel I bzw. Formel IIA1 oder IIA2 mit Resten wie oben beschrieben mit einem kosmetisch oder dermatologisch oder für Nahrungsmittel geeigneten Träger vermischt wird, und die Verwendung einer Verbindung der Formel I bzw. Formel IIA1 oder IIA2 zur Herstellung einer Zubereitung.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß Formel I bzw. Formel IIA1 oder IIA2 in dem Träger zur Folge haben.

Es wurde auch festgestellt, dass Verbindungen der Formel I bzw. Formel IIA1 oder IIA2 stabilisierend auf die Zubereitung wirken können. Bei der Verwendung in entsprechenden Produkten bleiben diese daher auch länger stabil und verändern ihr Aussehen nicht. Insbesondere bleibt auch bei längendauernder Anwendung bzw. längerer Lagerung die Wirksamkeit der Inhaltsstoffe, z.B. Vitamine, erhalten. Dies ist unter anderem besonders vorteilhaft bei Zusammensetzungen zum Schutz der Haut gegen die Einwirkung von UV-Strahlen, da diese Kosmetika besonders hohen Belastungen durch die UV-Strahlung ausgesetzt sind.

Die positiven Wirkungen von Verbindungen der Formel I bzw. Formel IIA1 oder IIA2 ergeben deren besondere Eignung zur Verwendung in kosmetischen oder pharmazeutischen Zubereitungen.

Ebenso positiv sind die Eigenschaften von Verbindungen mit der Formel I bzw. Formel IIA1 oder IIA2 zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel I bzw. Formel IIA1 oder IIA2 angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen).
Weitere Gegenstände der vorliegenden Erfindung sind dementsprechend die Verwendung einer Verbindung nach Formel I bzw. Formel IIA1 oder IIA2 als Nahrungsmittelzusatz für die human- oder Tierernährung sowie Zubereitungen, die Nahrungsmittel oder Nahrungsergänzungsmittel sind und entsprechende Träger enthalten.

Nahrungsmittel, die nach der vorliegenden Erfindung mit einer oder mehreren Verbindungen der Formel I bzw. Formel IIA1 oder IIA2 angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel, die nach der vorliegenden Erfindung mit einer oder mehreren Verbindungen der Formel I bzw. Formel IIA1 oder IIA2 angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel I bzw. Formel IIA1 oder IIA2 angereichert zu werden, beispielsweise Multivitaminpräparate, Mineralstoffmischungen oder gezuckerter Saft. Als weitere Beispiele für Nahrungsmittel, die nach der vorliegenden Erfindung mit einer oder mehreren Verbindungen der Formel I bzw. Formel IIA1 oder IIA2 angereichert werden können, seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel I bzw. Formel IIA1 oder IIA2 angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formel I bzw. Formel IIA1 oder IIA2 angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel I bzw. Formel IIA1 oder IIA2 angereicherten erfindungsgemäßen Nahrungsmittel können mit Hilfe von Techniken hergestellt werden, die dem Fachmann wohl bekannt sind.

Durch ihre Wirkung eignen sich Verbindungen der Formel I bzw. Formel IIA1 oder IIA2 auch als Arzneimittelinhaltsstoff. Verbindungen der Formel I bzw. Formel IIA1 oder IIA2 können beispielsweise zur vorbeugenden Behandlungen von Entzündungen und Allergien der Haut sowie in bestimmten Fällen zur Verhütung bestimmter Krebsarten verwendet werden. Insbesondere eignen sich Verbindungen der Formel I bzw. Formel IIA1 oder IIA2 zur Herstellung eines Arzneimittels zur Behandlung von Entzündungen, Allergien und Irritationen, insbesondere der Haut. Ferner können Arzneimittel hergestellt werden in einer Wirkung als Venentonikum, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Hautelastizität und als Antialterungsmittel. Weiter zeigen in diesem Zusammenhang bevorzugte Verbindungen der Formel I bzw. Formel IIA1 oder IIA2 antiallergische und anti-inflammatorische und antiirritative Wirkungen. Sie eignen sich daher zur Herstellung von Arzneimitteln zur Behandlung von Entzündungen oder allergischen Reaktionen.

Bevorzugte entzündungshemmende Zubereitungen können dabei mindestens einen zusätzlichen entzündungshemmenden Wirkstoff, der vorzugsweise ausgewählt ist aus den Glukokortikoiden oder Tacrolimus enthalten.

Tacrolimus wurde aus dem Pilz Streptomyces tsukukaensis isoliert und zeigt immunsuppresive Wirkung.

Geeignete Glukokortikoide sind beispielsweise Prednison, Cloprednol, Triamcinolon, Methylprednisolon, Dexamethason, Betamethason, Desoximetason, Clobetasonbutyrat, Halcinonid, Clobetasolpropionat, Prednisolon, Hydrocortisonbutyrat, Betamethasondipropionat, Fluocinolonacetonid, Fluocinolonacetonid, Betamethasonvalerat, Hydrocortison (Cortisol), Cortisonacetat, Prednicarbat Diflucortolonvalerat, Triamcinolonacetonid, Fluocinolonacetonid, Fluocortolon und Fluocortolon-21-hexanoat.

Derartige Zubereitungen, die eine Wirkstoffkombination aus mindestens einer Komplex-Verbindung der Formel I bzw. mindestens eine Verbindung nach Formel IIA1 oder IIA2 und mindestens ein Cyclodextrin mit mindestens einem der oben angegebenen weiteren entzündungshemmenden Wirkstoffen enthalten, zeigen eine besonders starke entzündungshemmende Wirkung.

Insbesondere hat sich gezeigt, dass die Komplex-Verbindungen der Formel I und die erfindungsgemäßen Zubereitungen sich besonders vorteilhaft in der Behandlung atopischer Ekzeme, wie insbesondere Milchschorf, Neurodermitis, Prurigo und Dermatitis sicca einsetzen lassen.
Dabei hat sich gezeigt, dass sie

- die akuten Symptome stark vermindern können,
- die Häufigkeit des Auftretens von akuten Symptomen reduzieren können,
- allgemein zur Verbesserung des Hautbildes beitragen.

Die Zubereitungen enthaltend eine oder mehrere Verbindungen der Formel I eignen sich auch zum Schutz menschlicher Haut bzw. zum Schutz von Körperzellen gegen oxidativen Stress, d.h. z.B. gegen Schädigungen durch Radikale, wie sie z.B. durch Sonneneinstrahlung, Wärme oder andere Einflüsse erzeugt werden. Die Zubereitungen enthaltend eine oder mehrere Verbindungen der Formel I sind insbesondere zur Verringerung der Hautalterung geeignet.

Gegenstand der vorliegenden Erfindung ist somit auch die Verwendung von einer oder mehreren Verbindungen der Formel I als Wirkstoff zum Schutz gegen oxidativen Stress. Ein weiterer Gegenstand der vorliegenden Erfindung ist zudem die Verwendung von einer oder mehreren Verbindungen der Formel I zur Vermeidung der Hautalterung.

Die Verbindungen der Formel I besitzen anti-allergische, anti-inflammatorische, entzündungshemmende und anti-irritative Eigenschaften und können somit zur Behandlung oder vorbeugenden Behandlung von Allergien, Entzündungen und Irritationen, insbesondere der Haut, verwendet werden. Ein weiterer Gegenstand der vorliegenden Erfindung ist daher die Verwendung von einer oder mehreren Verbindungen der Formel I als Wirkstoff mit anti-allergischer, anti-inflammatorischer, entzündungshemmender und anti-irritativer Wirkung.

Erfindungsgemäß bevorzugte Verwendungen der Verbindungen gemäß Formel I bzw. von Zubereitungen enthaltend mindestens eine Verbindung nach Formel I sind dabei insbesondere die Verwendung zur Prophylaxe gegen zeit- und/oder lichtinduzierte Alterungsprozesse der menschlichen Haut oder menschlicher Haare, insbesondere zur Prophylaxe gegen trockene Haut, Faltenbildung und/oder Pigmentstörungen, und/oder zur Reduktion oder Verhinderung schädigender Effekte von UV-Strahlen auf die Haut, sowie zur Prophylaxe gegen oder Reduktion von Hautunebenheiten, wie Falten, feinen Linien, rauher Haut oder großporiger Haut.

Weisen die erfindungsgemäß einzusetzenden Verbindungen freie Hydroxy-Gruppen auf, so zeigen sie neben den beschriebenen Eigenschaften zusätzlich eine Wirkung als Antioxidans und/oder Radikalfänger. Bevorzugt sind daher auch Zubereitungen mit Lichtschutzeigenschaften enthaltend zumindest eine Verbindung der Formel I, die dadurch gekennzeichnet ist, dass mindestens einer der Reste $R^1$ bis $R^3$ steht für OH, wobei vorzugsweise mindestens einer der Reste $R^1$ oder $R^2$ für OH steht.

Damit die Verbindungen der Formel I ihre positive Wirkung als Radikalfänger auf die Haut besonders gut entwickeln können, kann es bevorzugt sein die Verbindungen der Formel I in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar.
Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.
Es wird vermutet, dass bevorzugte Verbindungen der Formel I auch als Enzymhemmer wirken. Sie hemmen vermutlich Histidindecarboxylase,
Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der Thrombozytenaggregation aufweisen.

Aufgrund dieser Eigenschaften eignen sich die erfindungsgemäßen Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse. Alle diese Verwendungen bzw. die Verwendung der Verbindungen der Formel I zur Herstellung entsprechend einsetzbarer Zubereitungen sind ausdrücklich auch Gegenstand der vorliegenden Erfindung.

Insbesondere eignen sich bevorzugte erfindungsgemäße Zusammensetzungen auch zur Behandlung von Hautkrankheiten, die mit einer Störung der Keratinisierung verbunden sind, die die Differenzierung und Zellprolif-eration betrifft, insbesondere zur Behandlung der Akne vulgaris, Akne comedonicá, der polymorphen Akne, der Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma basocellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der licht-bedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder

auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immuno-logischen Komponente, zur Behandlung von Herz-/Kreislauf-Erkran-kungen, wie Arteriosklerose oder Bluthochdruck, sowie des Insulin-unabhängigen Diabetes, zur Behandlung von Hautproblemen, die durch UV-Strahlung hervorgerufen werden.

Desweiteren besitzen Verbindungen der Formel I nur eine schwach ausgeprägte Eigenfarbe. Die schwach ausgeprägte Eigenfarbe ist z.B. dann von großem Vorteil, wenn in den Produkten eine Eigenfarbe der Inhaltsstoffe aus ästhetischen Gründen unerwünscht ist.

Der Anteil der Verbindungen der Formel I in der Zubereitung beträgt vorzugsweise von 0,01 bis 20 Gew.%, besonders bevorzugt von 0,05 bis 10 Gew.% und insbesondere bevorzugt von 0,1 bis 5 Gew.% bezogen auf die gesamte Zubereitung. Ganz außerordentlich bevorzugt beträgt der Anteil der Verbindungen der Formel I in der Zubereitung von 0,1 bis 2 Gew.% bezogen auf die gesamte Zubereitung.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die folgenden Beispiele sollen die vorliegende Erfindung verdeutlichen. Sie sind jedoch keinesfalls als limitierend zu betrachten. Alle Verbindungen oder Komponenten, die in den Zubereitungen verwendet werden können, sind entweder bekannt und käuflich erhältlich oder können nach bekannten Methoden synthetisiert werden. Die INCI-Namen der verwendeten Rohstoffe sind wie folgt (die INCI-Namen werden definitionsgemäß in Englischer Sprache angegeben):

## Beispiele

**Liste der eingesetzten Rohstoffe**

| Rohstoff | INCI-Name |
|---|---|
| Abil WE 09 | Polyglyceryl-4-Isostearate, Cetyl Dimethicone Copolyol, Hexyl Laurate |
| Antaron V-220 | PVP/Eicosene Copolymer |
| Arlacel 80 | Sorbitan Oleate |
| Arlacel 165 V | Glyceryl Stearate, PEG-100 Stearate |
| Avocadoöl | Persea Gratissima |
| Bienenwachs | Beeswax |
| Biobase™ EP | Glyceryl Stearate, Cetearyl Alcohol, Sodium Stearoyl Lactylate, Lecithin |
| Carbopol ETD 2050 | Carbomer |
| Cetiol V | Decyl Oleate |
| Cetylalkohol | Cetyl Alcohol |
| Cetylisononanoat | Cetyl Isononanoate |
| Cutina HR | Hydrogenated Castor Oil |
| Dimeticon | Dimethicone |
| Eusolex®232 | Phenylbenzimidazole Sulfonic Acid |
| Eusolex® 2292 | Octyl Methoxycinnamate, BHT |
| Eusolex® 6300 | 4-Methylbenzylidene Camphor |
| Eusolex 8300 | 4-Methylbenzylidene |
| Eusolex® 9020 | Butyl Methoxydibenzoylmethane |
| Eusolex®HMS | Homosalate |
| Eusolex T-Aqua | Aqua (Water), Titanium Dioxide, Alumina, Sodium Metaphosphate, Phenoxyethanol, Sodium Methyl-paraben |
| Eutanol G | Octyldodecanol |
| Germaben II | Propylene Glycol, Diazolidinyl Urea, Methylparaben, Propylparaben |
| Germaben II-E | Propylene Glycol, Diazolidinyl Urea, Methylparaben, Propylparaben |
| Glycerin | Glycerin |

(fortgesetzt)

| Rohstoff | INCI-Name |
|---|---|
| Glycerin (87%) | Glycerin |
| Glycerin (87% reinst) | Glycerin |
| Glycerin, wasserfrei | Glycerin |
| Hetester PHA | Propylene Glycol Isoceteth-3 Acetate |
| Hexyllaurat | Hexyl Laurate |
| Imwitor 960 K Schuppen | Glyceryl Stearate SE |
| Isolan PDI | Diisostearoyl Polyglyceryl-3-Diisostearat |
| Isopropylmyristat | Isopropyl Myristate |
| Isopropylpalmitat | Isopropyl Palmitate |
| Jojobaöl | Buxus Chinensis (Jojoba Oil) |
| Karion F flüssig | Sorbitol |
| Keltrol RD | Xanthan Gum |
| Magnesiumsulfat | Magnesium Sulfate |
| Magnesiumsulfat-Heptahydrat | Magnesium Sulfate |
| Methyl-4-hydroxybenzoat | Methylparaben |
| Miglyol 812 | Caprylic/Capric Triglyceride |
| Miglyol 812 N | Caprylic/Capric Triglyceride |
| Miglyol 812, Neutralöl | Caprylic/Capric Triglyceride |
| Mirasil CM5 | Cyclomethicone |
| Mirasil DM 350 | Dimethicone |
| Montanov 68 | Cetearyl Alcohol, Cetearyl Glucoside |
| Natriumchlorid | Sodium Chloride |
| Natronlauge, 10%ig | Sodium Hydroxide |
| Oxynex®K | PEG-8, Tocopherol, Ascorbyl Palmitate, Ascorbic Acid, Citric Acid |
| Panthenol-D | Panthenol |
| Paracera M | Microwax |
| Paraffinöl, fl. | Mineral Oil |
| Parfümöl TND-2417 | Parfum |
| Pemulen TR-1 | Acrylates/$C_{10-30}$ Alkyl Acrylate Crosspolymer |
| Pemulen® TR-2 | Acrylates/$C_{10-30}$ Alkyl Acrylate Crosspolymer |
| Performa® V 825 | Synthetic Wax |
| Polyglyceryl-2-Dipolyhydroxystearat | Polyglyceryl-2 Dipolyhydroxystearate |
| Prisorine 2021 | Isopropyl Isostearate |
| Propandiol-1,2 | Propylene Glycol |
| Propyl-4-hydroxybenzoat | Propylparaben |
| Rhodicare S | Xanthan Gum |
| RonaCare™ ASC III | Aqua, Lecithin, Dipalmitoyl Hydroxyproline, Phenoxyethanol, Tall Oil Sterol, Linoleic Acid, Tocopherol, Sodium Ascorbate, Mannitol, Methylparaben, Ethylparaben, Propylparaben, Butylparaben |
| RonaCare™ Bisabolol | Bisabolol |
| RonaCare™ Ectoin | Ectoin |
| RonaCare™ LPO | Lauryl p-Cresol Ketoxime |
| RonaCare™ Tocopherolacetat | Tocopheryl Acetate |
| Sepigel 305 | Polyacrylamide, $C_{13-14}$ Isoparaffin, Laureth-7 |
| SFE 839 | Cyclopentasiloxane, Dimethicone/ Vinyldimethicone Crosspolymer |
| Shea Butter | Shea Butter |
| Steareth-2 | Steareth-2 |
| Steareth-10 | Steareth-10 |
| Stearinsäure | Stearic Acid |
| DL-α-Tocopherolacetat | Tocopherol Acetate |

(fortgesetzt)

| Rohstoff | INCI-Name |
|---|---|
| Triethanolamin | Triethanolamine |
| Triethanolamin reinst | Triethanolamine |
| Wasser, demineralisiert | Aqua (Water) |
| Zinkstearat | Zinc Stearate |

**Beispiel A - Herstellung eines Tilirosid - Cyclodextrin-Komplexes**

0,9 g Hydroxypropyl-gamma-cyclodextrin (Fa. Aldrich; 2'-Hydroxypropylcyclooctaamylose; Cas.-Nr. 128446-34-4) werden in 8 ml Wasser vorgelegt und auf 50°C erwärmt. 0,15 g Tilirosid werden in 8 ml Ethanol bei Raumtemperatur gelöst und in die vorgelegte Lösung zugetropft. Die Lösung wird drei Tage bei 50°C gerührt. Das Ethanol wird aus der Lösung abdestilliert. Der Rückstand wird im Vakuum bis zur Trockne eingeengt und der verbleibende gelbe Feststoff bei 40°C und 200 mbar über Nacht nachgetrocknet. Ausbeute: 1,02g = 97,2 % der Theorie gelber kristalliner Rückstand.

**Charakterisierung:**

**- Nachweis der Komplexbildung mittels 2D- NMR Spekrum**

ROESY Spektren zeigen Wechselwirkung von räumlich benachbarten Atomen auf. Räumlich nahe stehende Atome geben im ROESY 2D-NMR Spekrum signale. Hier wurde der Komplex mittels ROESY vermessen um aufzuklären über welche Molekülbestandteile des Tilirosids die Komplexbildung erfolgt.
Im ROESY Spektrum (Lösungsmittel $D_2O$) treten Signale auf die einer Wechselwirkung der Tilirosid Atome 2''', 3''', 6 und 8 (vgl. Formelzeichnung) mit den Cyclodextrin-Molekülen zugeordnet werden können.

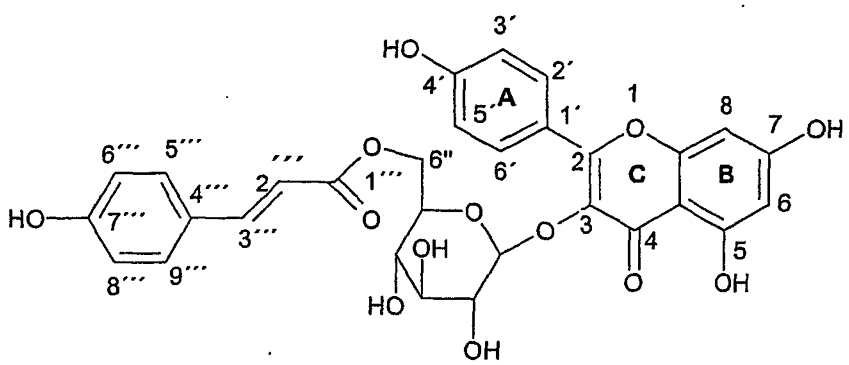

Die NMR-Daten passen zu der Interpretation, dass ein Komplex entstanden ist, der aus Tilirosid und zwei Cyclodextrinmolekülen besteht, die räumlich am aromatischen Ring der Cumarsäure bzw. am B Ring des Flavongrundkörpers angeordnet sind.

**- Gehalt Tilirosid im Feststoff (HPLC- Bestimmung)**

8,0 mg Tilirosid werden in 3 ml Methanol und 1 ml THF gelöst und im Messkolben mit Eluent (Acetonil/$H_2O$ 2/8) auf 10,0 ml gefüllt. (Peakfläche von 12783970).
11,5 mg Komplex werden in 3 ml Methanol und 1 ml Tetrahydrofuran gelöst und im Messkolben mit Eluent (Acetonitril/$H_2O$ 2/8) auf 10,0 ml gefüllt. (Peakfläche Tilirosid 2503977).
Berechnung: 8,0mg Tilirosid ergeben eine Fläche von 12783970 11,5mg Tilirosid müssten eine Fläche von 18376957 ergeben. Gefunden wird eine Fläche von 2503977.
Daraus folgt: Der Komplex besteht zu 13,6 Gew.-% aus Tilirosid. In dem Komplex liegt ein Massenverhältnis Tilirosid : Cyclodextrin von 13,6 : 86,4 vor. Dies entspricht einem Molverhältnis von 1:2 (Theoretisches Massenverhältnis des Tilirosid $(Cyclodextrin)_2$ Komplexes = 14,4 : 85,6). Bei der Komplexverbindung handelt es sich um einen Komplex [Tili-

rosid][ Hydroxypropyl-gamma-cyclodextrin]$_2$.

**Löslichkeit des Tilirosid-Cyclodextrin Komplexes:**

In 1 ml Wasser wird 0,5 g Komplex gelöst, ohne Sättigung zu erreichen. Das enspricht einer Löslichkeit bezogen auf reines Tilirosid von mindestens 72 mg/ml.

In den folgenden Beispielrezepturen 1 bis 5 wird Tilirosid jeweils als Tilirosid-Hydroxypropyl-gamma-cyclodextrin-Komplex gemäß Beispiel A eingesetzt.

**Beispiel 1**

**Lotion (W/O) zum Auftragen auf die Haut**

| | | Gew.% |
|---|---|---|
| A | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | Tilirosid | 0,5 |
| B | Glycerin | 5,0 |
| | Magnesiumsulfat-Heptahydrat | 1,0 |
| | Konservierungsmittel | q.s. |
| | Wasser, demineralisiert | ad 100 |

Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:

0,05 % Propyl-4-hydroxybenzoat
0,15 % Methyl-4-hydroxybenzoat

**Beispiel 2**

**Lotion (W/O) zum Auftragen auf die Haut**

| | | Gew.% |
|---|---|---|
| A | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| B | sulfatisiertes Tilirosid, Natriumsalz (Beispiel A) | 1,0 |

(fortgesetzt)

| | Gew.% |
|---|---|
| Glycerin | 5,0 |
| Magnesiumsulfat-Heptahydrat | 1,0 |
| Konservierungsmittel | q.s. |
| Wasser, demineralisiert | ad 100 |

Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:

0,05 % Propyl-4-hydroxybenzoat
0,15 % Methyl-4-hydroxybenzoat

**Beispiel 3**

**Lotion (W/O) zum Auftragen auf die Haut**

| | | Gew.% |
|---|---|---|
| **A** | 4,6,3',4'-Tetrahydroxybenzylcoumaranon-3 | 1,0 |
| | Polyglyceryl-2-Dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | Tilirosid | 1,0 |
| **B** | Glycerin | 5,0 |
| | Magnesiumsulfat-Heptahydrat | 1,0 |
| | Konservierungsmittel | q.s. |
| | Wasser, demineralisiert | ad 100 |

Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:

0,05 % Propyl-4-hydroxybenzoat
0,15 % Methyl-4-hydroxybenzoat

**Beispiel 4**

Aus folgenden Komponenten wird eine Creme (O/W), enthaltend Ectoin, hergestellt:

| | | | Gew.% |
|---|---|---|---|
| **A** | Paraffin, dünnflüssig | (1) | 8,0 |

(fortgesetzt)

| | | | Gew.% |
|---|---|---|---|
| | Isopropylmyristat | (1) | 4,0 |
| | Mirasil CM5 | (2) | 3,0 |
| | Stearinsäure | (1) | 3,0 |
| | Arlacel 165 V | (3) | 5,0 |
| | Tilirosid | | 1,0 |
| **B** | Glycerin (87%) | (1) | 3,0 |
| | Germaben II | (4) | 0,5 |
| | Wasser, demineralisiert | | ad 100 |
| **C** | RonaCare™ Ectoin | (1) | 1,0 |

Herstellung

Zunächst werden die Phasen A und B getrennt auf 75°C erwärmt. Danach wird Phase A unter Rühren langsam zu Phase B gegeben und solange gerührt, bis eine homogene Mischung entsteht. Nach Homogenisierung der Emulsion wird unter Rühren auf 30°C abgekühlt. Anschließend wird auf 35°C erwärmt, die Phase C zugegeben und bis zur Homogenität gerührt.

Bezugsquellen

(1) Merck KGaA
(2) Rhodia
(3) Uniqema
(4) ISP

**Beispiel 5**

**Topische Zusammensetzung als W/O-Emulsion**

| | | | Gew.% |
|---|---|---|---|
| **A** | Isolan PDI | (2) | 3,0 |
| | Paraffinöl, fl. | (1) | 17,0 |
| | Isopropylmyristat | | 5,0 |
| | Bienenwachs | | 0,2 |
| | Cutina HR | (2) | 0,3 |
| | Tilirosid | | 1,0 |
| **B** | Wasser, demineralisiert | | ad 100 |
| | Glycerin (87%) | | 4,0 |
| | Magnesiumsulfat | | 1,0 |
| | Germaben II-E | (3) | 1,0 |
| **C** | RonaCare™ LPO | (1) | 2,0 |

Herstellung

Die Phasen A und B werden auf 75°C erwärmt. Phase B wird unter Rühren zu Phase A gegeben. Anschließend wird das Gemisch bei 9000upm 2 Min. mit dem Turrax homogenisiert. Das erhaltene Gemisch wird auf 30 bis 35°C

abgekühlt, und C wird eingerührt.

Bezugsquellen

(1) Merck KGaA
(2) Goldschmidt AG
(3) ISP

**Beispiel 6: Zubereitungen**

Im folgenden werden beispielhaft Rezepturen für kosmetische Zubereitungen angegeben, die den Tilirosid-(2-Hydroxypropyl-gamma-Cyclodextrin)-Komplex (= Tilirosid-CD) nach Beispielen A enthalten. Im übrigen sind die INCI-Bezeichnungen der handelsüblichen Verbindungen angegeben.

UV-Pearl , OMC steht für die Zubereitung mit der INCI-Bezeichnung:

Water (for EU: Aqua), Ethylhexyl Methoxycinnamate, Silica, PVP, Chlorphenesin, BHT; diese Zubereitung ist im Handel unter der Bezeichnung Eusolex®UV Pearl™OMC von der Merck KGaA, Darmstadt erhältlich.
Die anderen in den Tabellen angegebenen UV-Pearl sind jeweils analog zusammengesetzt, wobei OMC gegen die angegebenen UV-Filter ausgetauscht wurde.

Tabelle 1 W/O-Emulsionen (Zahlen in Gew.-%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Titanium dioxide | | 2 | 5 | | | | | | | 3 |
| Tilirosd-CD | 5 | 3 | 2 | 1 | 2 | 1 | 2 | 1 | 1 | 1 |
| Zinc oxide | | | | | | | | 5 | 2 | |
| UV-Pearl, OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Water | ad 100 | ad 100 | ad 100 | ad 100 | ad 100 | ad 100 | ad 100 | ad 100 | ad 100 | ad 100 |

| | 1-11 | 1-12 | 1-13 | 1-14 | 1-15 | 1-16 | 1-17 | 1-18 |
|---|---|---|---|---|---|---|---|---|
| Titanium dioxide | 3 | | 2 | | 3 | | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| Tilirosid-CD | 1 | 3 | 2 | 5 | 1 | 3 | 7 | 2 |

(fortgesetzt)

| | 1-11 | 1-12 | 1-13 | 1-14 | 1-15 | 1-16 | 1-17 | 1-18 |
|---|---|---|---|---|---|---|---|---|
| Polyglyceryl-3-Dimerate | 3 | 3 | 3 | 3 | | | | |
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Dicocoyl Pentyerythrityl Citrate (and) Sorbitan Sesquioleate (and) Cera Alba (and) Aluminium Stearate | | | | | 6 | 6 | 6 | 6 |
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |
| Glycerin | | | | | 5 | 5 | 5 | 5 |
| Allantoin | | | | | 0,2 | 0,2 | 0,2 | 0,2 |
| Water | ad 100 | ad 100 | ad 100 | ad 100 | ad 100 | ad 100 | ad 100 | ad 100 |

(fortgesetzt)

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| Titanium dioxide | | 2 | 5 | | | | | | | 3 | 3 |
| Benzylidene malonate polysiloxane | | | | 1 | | | | | 1 | 1 | |
| Zinc oxide | | | | | | | | 5 | 2 | | |
| Tilirosid-CD | 5 | 5 | 5 | 5 | 7 | 5 | 5 | 5 | 5 | 5 | 8 |
| UV-Pearl, OCR | | 10 | | | | | | | | | 5 |
| UV-Pearl, EthylhexylDimethylPABA | | | 10 | | | | | | | | |
| UV-Pearl, Homosalate | | | | 10 | | | | | | | |
| UV-Pearl, Ethylhexyl salicylate | | | | | 10 | | | | | | |
| UV-Pearl , OMC, BP-3 | | | | | | 10 | | | | | |
| UV-Pearl , OCR, BP-3 | | | | | | | 10 | | | | |
| UV-Pearl, Ethylhexyl Dimethyl PABA, BP-3 | | | | | | | | 10 | | | |
| UV-Pearl, Homosalate, BP-3 | | | | | | | | | 10 | | |
| UV-Pearl, Ethylhexyl salicylate, BP-3 | | | | | | | | | | 10 | |
| BMDBM | | | | | | | | | | | 2 |
| UV-Pearl OMC, 4-Methylbenzylidene Camphor | 25 | | | | | | | | | | |
| Polyglyceryl-3-Dimerate | 3 | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 | 7 |

(fortgesetzt)

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| Caprylic/Capric Triglyceride | 7 | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 | 0,15 |
| Water | ad 100 | | | | | | | | | | |

Tabelle 2: O/W-Emulsionen, Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | | 2 | 5 | | | | | | | 3 |
| Methylene Bis-Benztriazolyl Tetramethylbutylphenol | | | | | | 1 | 2 | 1 | | |
| 2-(1-Ethyl-hexyl)-5,7-dihydroxy-chromen-4-on | | | | 1 | 2 | | | | 1 | 1 |
| 4'-Methoxy-6-hydroxyflavon | 1 | 3 | | 2 | | 5 | | 5 | 2 | |
| Tilirosid-CD | 5 | 5 | 5 | 5 | 5 | 5 | 5 | 5 | 5 | 5 |
| 2-Carboxyl-5,7-dihydroxy-chromen-4-on | 1 | 5 | 4 | | 6 | | 7 | | 2 | 1 |
| 4-Methylbenzyliden Camphor | 2 | | 3 | | 4 | | 3 | | 2 | |
| BMDBM | 1 | 3 | | 3 | 3 | | 3 | 3 | 3 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Glyceryl Stearate | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11,5 | 11,5 | 11,5 | 11,5 | 11,5 | 11,5 | 11,5 | 11,5 | 11,5 | 11,5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Glyceryl Stearate SE | | | | | | | | | | |
| Stearic Acid | | | | | | | | | | |
| Persea Gratissima | | | | | | | | | | |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Glycerin | | | | | | | | | | |
| Water | ad 100 | ad 100 | ad 100 | ad 100 | ad 100 | ad 100 | ad 100 | ad 100 | ad 100 | ad 100 |
| | | | | | | | | | | |
| | | | 2-11 | 2-12 | 2-13 | 2-14 | 2-15 | 2-16 | 2-17 | 2-18 |
| Titanium dioxide | | | 3 | 2 | | | | | 2 | 5 |

(fortgesetzt)

| | 2-11 | 2-12 | 2-13 | 2-14 | 2-15 | 2-16 | 2-17 | 2-18 |
|---|---|---|---|---|---|---|---|---|
| Benzylidene malonate polysiloxane | | 1 | 0,5 | | | | | |
| Methylene Bis-Benztriazolyl Tetramethylbutylphenol | 1 | 1 | 0,5 | | | | | |
| 4'-Methoxy-7-β-glucosidflavon | | | | 1 | 2 | | | |
| Tilirosid-CD | 1 | 3 | 0,1 | 2 | 0,5 | 5 | 0,2 | 5 |
| 2-Carboxyl-7-hydroxy-chromen-4-on | 5 | 5 | 5 | 5 | 5 | 5 | 5 | 5 |
| Ectoin | 1 | 5 | 4 | | 6 | | 7 | |
| Zinc oxide | | | 2 | | | | | |
| UV-Pearl, OMC | 15 | 15 | 15 | 30 | 30 | 30 | 15 | 15 |
| 4-Methylbenzyliden Camphor | | | | 3 | | | | |
| BMDBM | | | | 1 | | | | |
| Phenylbenzimidazole Sulfonic Acid | | | | | 4 | | | |
| Stearyl Alcohol (and) Steareth-7 (and) Steareth-10 | 3 | 3 | 3 | 3 | | | | |
| Glyceryl Stearate (and) Ceteth-20 | 3 | 3 | 3 | 3 | | | | |
| Glyceryl Stearate | 3 | 3 | 3 | 3 | | | | |
| Microwax | 1 | 1 | 1 | 1 | | | | |
| Cetearyl Octanoate | 11,5 | 11,5 | 11,5 | 11,5 | | | | |
| Caprylic/Capric Triglyceride | 6 | 6 | 6 | 6 | 14 | 14 | 14 | 14 |
| Oleyl Oleate | 6 | 6 | 6 | 6 | | | | |
| Propylene Glycol | 4 | 4 | 4 | 4 | | | | |
| Glyceryl Stearate SE | | | | | 6 | 6 | 6 | 6 |
| Stearic Acid | | | | | 2 | 2 | 2 | 2 |
| Persea Gratissima | | | | | 8 | 8 | 8 | 8 |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Tromethamine | | | | | 1,8 | | | |
| Glycerin | | | | | 3 | 3 | 3 | 3 |
| Water | ad 100 | ad 100 | ad 100 | ad 100 | ad 100 | ad 100 | ad 100 | ad 100 |

| | 2-19 | 2-20 | 2-21 | 2-22 | 2-23 | 2-24 | 2-25 | 2-26 | 2-27 | 2-28 |
|---|---|---|---|---|---|---|---|---|---|---|
| Titanium dioxide | | | | | | | 3 | 3 | | 2 |
| Benzylidene malonate polysiloxane | 1 | 2 | | | | 1 | 1 | | 1 | 0,5 |
| 7,8,3',4'-Tetrahydroxyflavon | | | | 1 | 2 | | | | 1 | 1 |
| Tilirosid-CD | 1 | 3 | 0,2 | 2 | 0,3 | 5 | 0,5 | 5 | 2 | 0,8 |

(fortgesetzt)

| | 2-19 | 2-20 | 2-21 | 2-22 | 2-23 | 2-24 | 2-25 | 2-26 | 2-27 | 2-28 |
|---|---|---|---|---|---|---|---|---|---|---|
| 2-Methyl-5,7-dihydroxy-chromen-4-on | 5 | 5 | 5 | 5 | 5 | 5 | 5 | 5 | 5 | 5 |
| Methylene Bis-Benztriazolyl Tetramethylbutylphenol | | | 1 | 2 | 1 | | | 1 | 1 | 0,5 |
| Zinc oxide | | | | | 5 | 2 | | | | 2 |
| UV-Pearl, OMC | 15 | 15 | 15 | 15 | 15 | 15 | 15 | 15 | 15 | 15 |
| Caprylic/Capric Triglyceride | 14 | 14 | 14 | 14 | 14 | 14 | 14 | 14 | 14 | 14 |
| Glyceryl Stearate SE | 6 | 6 | 6 | 6 | 6 | 6 | 6 | 6 | 6 | 6 |
| Stearic Acid | 2 | 2 | 2 | 2 | 2 | 2 | 2 | 2 | 2 | 2 |
| Persea Gratissima | 8 | 8 | 8 | 8 | 8 | 8 | 8 | 8 | 8 | 8 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Glycerin | 3 | 3 | 3 | 3 | 3 | 3 | 3 | 3 | 3 | 3 |
| Water | ad 100 | ad 100 | ad 100 | ad 100 | ad 100 | ad 100 | ad 100 | ad 100 | ad 100 | ad 100 |

Tabelle 3: Gele, Zahlen in Gew.-%

| | 3-1 | 3-2 | 3-3 | 3-4 | 3-5 | 3-6 | 3-7 | 3-8 | 3-9 | 3-10 |
|---|---|---|---|---|---|---|---|---|---|---|
| a = aqueaous gel | | | | | | | | | | |
| Titanium dioxide | | 2 | 5 | | | | | | | 3 |
| Tilirosid-CD | 1 | 3 | 0,2 | 2 | 0,5 | 5 | 1 | 5 | 2 | 1,5 |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl , Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Butylmethoxydibenzoylmethane | | 1 | | | | | | | | |
| Phenylbenzimidazole Sulfonic Acid | | | 4 | | | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |

(fortgesetzt)

| | 3-1 | 3-2 | 3-3 | 3-4 | 3-5 | 3-6 | 3-7 | 3-8 | 3-9 | 3-10 |
|---|---|---|---|---|---|---|---|---|---|---|
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

## Patentansprüche

**1.** Komplex-Verbindung nach Formel I

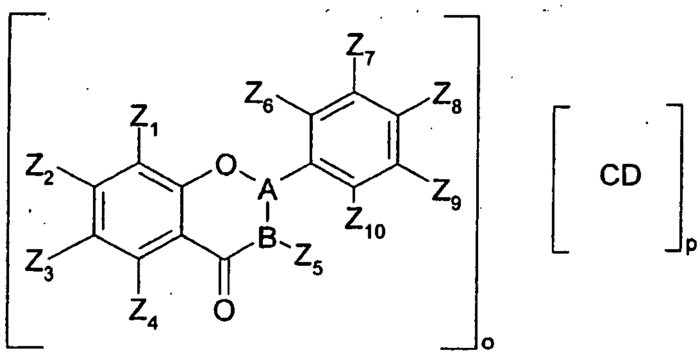

I

wobei es sich bei dem Flavonoid-Anteil

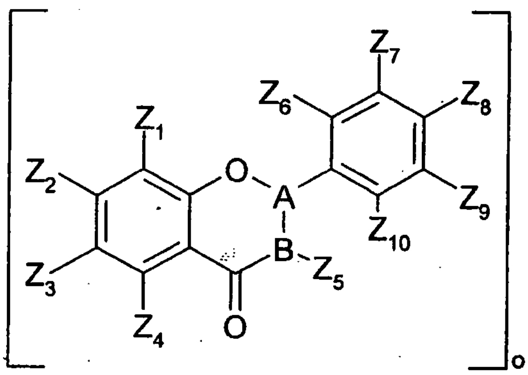

um eine Verbindung der Formel IIA1 oder IIA2 handelt

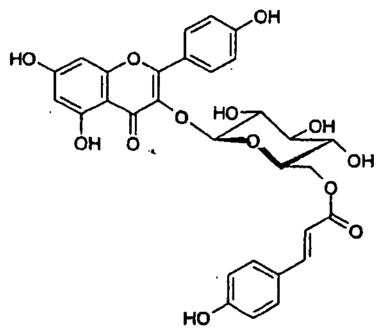

IIA1

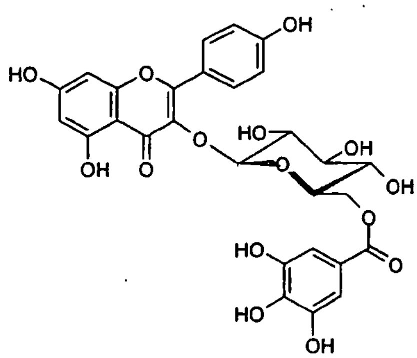

IIA2

o steht für die Zahl 1,
CD steht für ein gegebenenfalls an ein oder mehreren Hydroxygruppen $C_{1-24}$-Alkyl- oder $C_{1-24}$-Hydroxyalkyl-substituiertes gamma-Cyclodextrin und
p in dem Bereich 1,75 bis 2,1 liegt.

2. Komplex-Verbindung nach Anspruch 1, **dadurch gekennzeichnet, dass** CD Hydroxypropyl-gamma-cyclodextrin bedeutet.

3. Komplex-Verbindung nach mindestens einem der vorstehenden Ansprüche, **dadurch gekennzeichnet, dass** der Flavonoid-Anteil in der Form eines Pflanzenextrakts oder eines aufgereinigten Pflanzenextrakts oder in Form der aus dem Pflanzenextrakt hergestellten Reinsubstanz eingesetzt wird, wobei der Pflanzenextrakt 5 bis 90 Gew.% des Flavonoids gemäß Formel I enthält.

4. Komplex-Verbindung nach mindestens einem der vorstehenden Ansprüche, **dadurch gekennzeichnet, dass** in Formel I p gleich 2 ist.

5. Verfahren zur Herstellung von Komplex-Verbindungen nach mindestens einem der Ansprüche 1 bis 4, **dadurch gekennzeichnet, dass** Verbindungen gemäß Formel IIA1 oder IIA2
in Lösung mit Cyclodextrinen CD nach Anspruch 1 oder 2, vorzugsweise bei erhöhter Temperatur, umgesetzt werden.

6. Verfahren nach Anspruch 5, **dadurch gekennzeichnet, dass** das Cyclodextrin im Überschuß oder genau im Molverhältnis 2 : 1 bezogen auf das Flavonoid der Formel IIA1 oder IIA2 eingesetzt wird.

7. Zubereitung enthaltend einen geeigneten Träger, **dadurch gekennzeichnet, dass** die Zubereitung

- 0,005 bis 99 Gew.-% einer Komplex-Verbindung nach Formel I gemäß Anspruch 1 enthält.

8. Zubereitung nach Anspruch 7, **dadurch gekennzeichnet, dass** die eine oder die mehreren Verbindungen der Formel I in Mengen von 0,01 bis 20 Gew.%, vorzugsweise 0,05 bis 10 Gew.-% und insbesondere bevorzugt 0,1 bis

5 Gew.-% in der Zubereitung enthalten sind.

**9.** Zubereitung nach Anspruch 8-, **dadurch gekennzeichnet, dass** der Gehalt an Cyclodextrinen in der Zubereitung 0,01-20,0 Gew.%, bevorzugt 0,05-10,0 Gew.%, besonders bevorzugt 0,1-5,0 Gew.%, jeweils bezogen auf das Gesamtgewicht der Zusammensetzung beträgt und der Gehalt an Verbindungen der Formel IIA1 oder IIA2 in der Zubereitung von 0,01 bis 20 Gew.%, vorzugsweise 0,05 bis 10 Gew.% und insbesondere bevorzugt von 0,1 bis 5 Gew.% bezogen auf die gesamte Zubereitung, wobei der der Anteil der Verbindungen der Formel IIA1 oder IIA2 in der Zubereitung ganz außerordentlich bevorzugt im Bereich von 0,1 bis 2 Gew.% bezogen auf die gesamte Zubereitung liegt.

**10.** Zubereitung nach mindestens einem der vorstehenden Ansprüche, **dadurch gekennzeichnet, dass** die Zubereitung ein oder mehrere Antioxidantien und/oder einen oder mehrere UV-Filter enthält.

**11.** Zubereitung nach mindestens einem der vorstehenden Ansprüche, **dadurch gekennzeichnet, dass** die Zubereitung einen oder mehrere weitere Wirkstoffe mit hautpflegender und/oder entzündungshemmender Wirkung enthält, wobei es sich bei den ein oder mehreren weiteren Wirkstoffen vorzugsweise um Glukokortikoide oder um Tacrolimus handelt.

**12.** Verwendung einer Komplex-Verbindung gemäß Formel I nach Anspruch 1 oder einer Zubereitung nach mindestens einem der Ansprüche 7 bis 11 zur Herstellung eines Medikaments zur Prophylaxe und/oder Therapie von Ekzemen, insbesondere atopischer Ekzeme, wie insbesondere Milchschorf, Neurodermitis, Prurigo und Dermatitis sicca.

**13.** Nicht-medizinische Verwendung einer Komplex-Verbindung gemäß Formel I nach Anspruch 1 oder einer Zubereitung nach mindestens einem der Ansprüche 7 bis 11 zur Pflege, Konservierung oder Verbesserung des allgemeinen Zustandes der Haut oder Haare.

**14.** Nicht-medizinische Verwendung einer Komplex-Verbindung gemäß Formel I nach Anspruch 1 oder einer Zubereitung nach mindestens einem der Ansprüche 7 bis 11 zur Prophylaxe gegen zeit- und/oder lichtinduzierte Alterungsprozesse der menschlichen Haut oder menschlicher Haare, insbesondere zur Prophylaxe gegen trockene Haut, Faltenbildung und/oder Pigmentstörungen, und/oder zur Reduktion oder Verhinderung schädigender Effekte von UV-Strahlen auf die Haut.

**15.** Nicht-medizinische Verwendung einer Komplex-Verbindung gemäß Formel I nach Anspruch 1 oder einer Zubereitung nach mindestens einem der Ansprüche 7 bis 11 zur Prophylaxe gegen oder Reduktion von Hautunebenheiten, wie Falten, feinen Linien, rauher Haut oder großporiger Haut.

**16.** Verwendung einer Komplex-Verbindung gemäß Formel I nach Anspruch 1 oder einer Zubereitung nach mindestens einem der Ansprüche 7 bis 11 zur Herstellung eines Medikaments zur Prophylaxe und/oder Therapie von Entzündungen oder allergischen Reaktionen.

**Claims**

**1.** Complex compound of the formula I

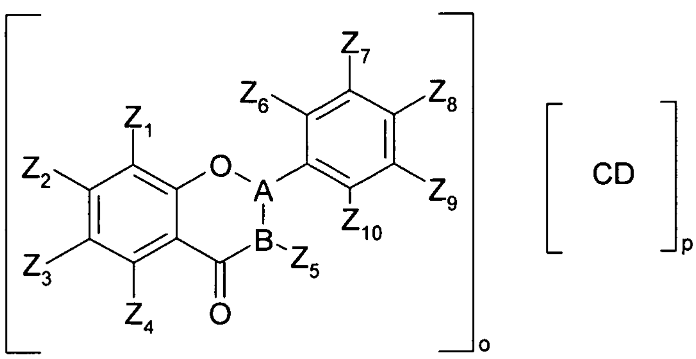

I

where the flavonoid moiety

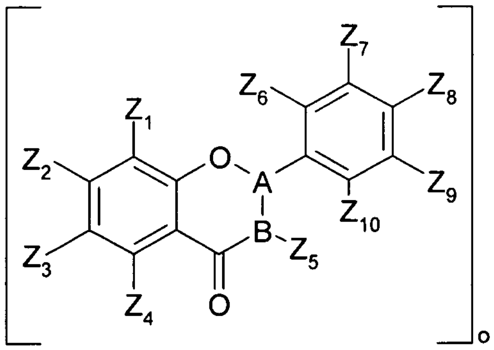

is a compound of the formula IIA1 or IIA2

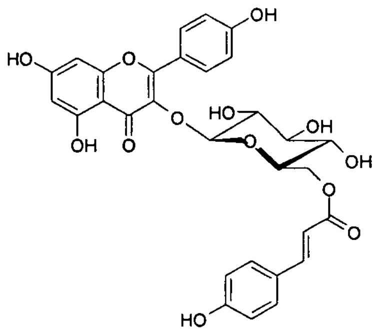

IIA1

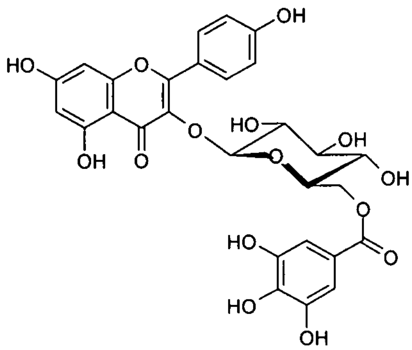

IIA2

o stands for the number 1,
CD stands for a gamma-cyclodextrin, which is optionally substituted by $C_{1-24}$-alkyl or $C_{1-24}$-hydroxyalkyl on one or more hydroxyl groups, and
p is in the range 1.75 to 2.1.

2. Complex compound according to Claim 1, **characterised in that** CD denotes hydroxypropyl-gamma-cyclodextrin.

3. Complex compound according to at least one of the preceding claims, **characterised in that** the flavonoid moiety is employed in the form of a plant extract or a purified plant extract or in the form of the pure substance prepared from the plant extract, where the plant extract comprises 5 to 90% by weight of the flavonoid of the formula I.

4. Complex compound according to at least one of the preceding claims, **characterised in that**, in the formula I, p is equal to 2.

5. Process for the preparation of complex compounds according to at least one of Claims 1 to 4, **characterised in that** compounds of the formula IIA1 or IIA2
are reacted in solution with cyclodextrins CD according to Claim 1 or 2, preferably at elevated temperature.

6. Process according to Claim 5, **characterised in that** the cyclodextrin is employed in excess or precisely in the molar ratio 2 : 1, based on the flavonoid of the formula IIA1 or IIA2.

7. Composition comprising a suitable vehicle, **characterised in that** the composition

- comprises 0.005 to 99% by weight of a complex compound of the formula I according to Claim 1.

8. Composition according to Claim 7, **characterised in that** the one or more compounds of the formula I are present in the composition in amounts of 0.01 to 20% by weight, preferably 0.05 to 10% by weight and particularly preferably 0.1 to 5% by weight.

9. Composition according to Claim 8, **characterised in that** the content of cyclodextrins in the composition is 0.01-20.0% by weight, preferably 0.05-10.0% by weight, particularly preferably 0.1-5.0% by weight, in each case based on the total weight of the composition, and the content of compounds of the formula IIA1 or IIA2 in the composition is from 0.01 to 20% by weight, preferably 0.05 to 10% by weight and particularly preferably from 0.1 to 5% by weight, based on the composition as a whole, where the proportion of the compounds of the formula IIA1 or IIA2 in the composition is very extraordinarily preferably in the range from 0.1 to 2% by weight, based on the composition as a whole.

10. Composition according to at least one of the preceding claims, **characterised in that** the composition comprises one or more antioxidants and/or one or more UV filters.

11. Composition according to at least one of the preceding claims, **characterised in that** the composition comprises one or more further active compounds having a skin-care and/or inflammation-inhibiting action, where the one or more further active compounds are preferably glucocorticoids or tacrolimus.

12. Use of a complex compound of the formula I according to Claim 1 or a composition according to at least one of Claims 7 to 11 for the preparation of a medicament for the prophylaxis and/or therapy of eczema, in particular atopic eczema, such as, in particular, milk crust, neurodermatitis, prurigo and dermatitis sicca.

13. Non-medical use of a complex compound of the formula I according to Claim 1 or a composition according to at least one of Claims 7 to 11 for the care, preservation or improvement of the general condition of the skin or hair.

14. Non-medical use of a complex compound of the formula I according to Claim 1 or a composition according to at least one of Claims 7 to 11 for prophylaxis against time- and/or light-induced ageing processes of the human skin or human hair, in particular for prophylaxis against dry skin, wrinkling and/or pigment defects, and/or for reducing or preventing damaging effects of UV rays on the skin.

15. Non-medical use of a complex compound of the formula I according to Claim 1 or a composition according to at least one of Claims 7 to 11 for prophylaxis against or reduction of skin unevenness, such as wrinkles, fine lines, rough skin or large-pored skin.

16. Use of a complex compound of the formula I according to Claim 1 or a composition according to at least one of Claims 7 to 11 for the preparation of a medicament for the prophylaxis and/or therapy of inflammation or allergic reactions.

**Revendications**

1. Composé complexe de formule I

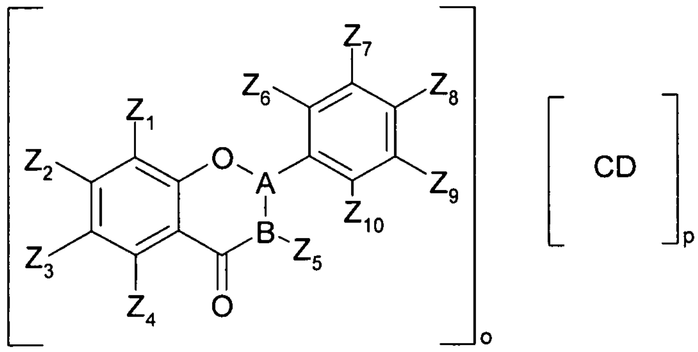

I

où le motif de flavonoïde

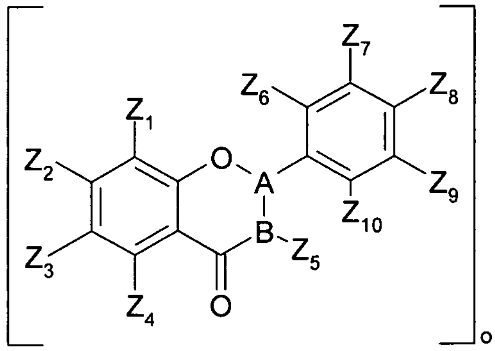

est un composé de formule IIA1 ou IIA2

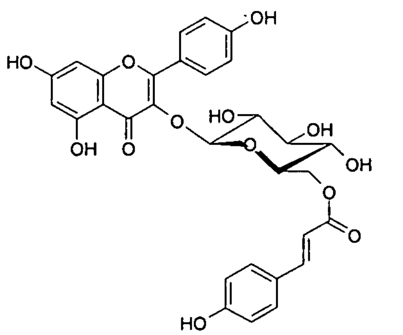

IIA1

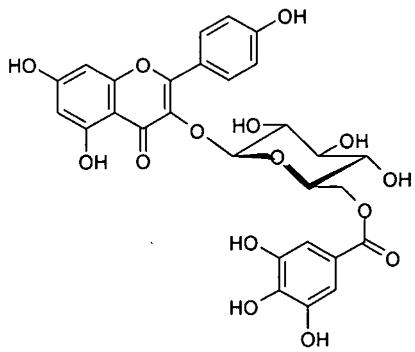

IIA2

o représente le chiffre 1,
CD représente une gamma-cyclodextrine, qui est éventuellement substituée par $C_{1-24}$-alkyle ou $C_{1-24}$-hydroxyalkyle sur un ou plusieurs groupements hydroxyle, et
p se trouve dans la plage allant de 1,75 à 2,1.

**2.** Composé complexe selon la revendication 1, **caractérisé en ce que** CD désigne hydroxypropyl-gamma-cyclodextrine.

**3.** Composé complexe selon au moins l'une des revendications précédentes, **caractérisé en ce que** le motif de flavonoïde est employé sous la forme d'un extrait végétal ou un extrait végétal purifié ou sous la forme de la substance pure préparée à partir de l'extrait végétal, où l'extrait végétal comprend de 5 à 90% en poids du flavonoïde de formule I.

**4.** Composé complexe selon au moins l'une des revendications précédentes, **caractérisé en ce que**, dans la formule I, p est égal à 2.

**5.** Procédé de préparation de composés complexes selon au moins l'une des revendications 1 à 4, **caractérisé en ce que** les composés de formule IIA1 ou IIA2 sont réagis en solution avec les cyclodextrines CD selon la revendication 1 ou 2, préférablement à une température élevée.

**6.** Procédé selon la revendication 5, **caractérisé en ce que** la cyclodextrine est employée en excès ou précisément selon le rapport molaire 2:1, sur la base du flavonoïde de formule IIA1 ou IIA2.

**7.** Composition comprenant un véhicule convenable, **caractérisée en ce que** la composition

- comprend de 0,005 à 99% en poids d'un composé complexe de formule I selon la revendication 1.

**8.** Composition selon la revendication 7, **caractérisée en ce que** les un ou plusieurs composés de formule I sont présents dans la composition en des quantités allant de 0,01 à 20% en poids, préférablement de 0,05 à 10% en poids et particulièrement préférablement de 0,1 à 5% en poids.

**9.** Composition selon la revendication 8, **caractérisée en ce que** la teneur en cyclodextrines dans la composition est de 0,01-20.0% en poids, préférablement de 0,05-10.0% en poids, particulièrement préférablement de 0,1-5,0% en poids, dans chaque cas sur la base du poids total de la composition, et la teneur en composés de formule IIA1 ou IIA2 dans la composition va de 0,01 à 20% en poids, préférablement de 0,05 à 10% en poids et particulièrement préférablement de 0,1 à 5% en poids, sur la base de la composition dans son ensemble, où la proportion des composés de formule IIA1 ou IIA2 dans la composition est très particulièrement préférablement dans la plage allant de 0,1 à 2% en poids, sur la base de la composition dans son ensemble.

**10.** Composition selon au moins l'une des revendications précédentes, **caractérisée en ce que** la composition comprend un ou plusieurs antioxydants et/ou un ou plusieurs filtres UV.

**11.** Composition selon au moins l'une des revendications précédentes, **caractérisée en ce que** la composition comprend un ou plusieurs autres composés actifs ayant une action de soin de la peau et/ou d'inhibition des inflammations, où les un ou plusieurs autres composés actifs sont préférablement des glucocorticoïdes ou du tacrolimus.

**12.** Utilisation d'un composé complexe de formule I selon la revendication 1 ou d'une composition selon au moins l'une des revendications 7 à 11 pour la préparation d'un médicament destiné à la prophylaxie et/ou la thérapie de l'eczéma, en particulier l'eczéma atopique, tel que, en particulier, le casque séborrhéique, la névrodermite, le prurigo et la dermite sèche.

**13.** Utilisation non médicale d'un composé complexe de formule I selon la revendication 1 ou d'une composition selon au moins l'une des revendications 7 à 11 pour le soin, la préservation ou l'amélioration de la condition générale de la peau ou des cheveux.

**14.** Utilisation non médicale d'un composé complexe de formule I selon la revendication 1 ou a composition selon au moins l'une des revendications 7 à 11 pour la prophylaxie contre les processus de vieillissement induits par le temps et/ou la lumière de la peau humaine ou des cheveux humains, en particulier pour la prophylaxie de la peau sèche, des rides et/ou des défauts de pigmentation, et/ou pour réduire ou empêcher les effets nuisibles des rayonnements UV sur la peau.

**15.** Utilisation non médicale d'un composé complexe de formule I selon la revendication 1 ou d'une composition selon au moins l'une des revendications 7 à 11 pour la prophylaxie contre ou la réduction des irrégularités de la peau, telles que les rides, les ridules, la peau rêche ou la peau à larges pores.

**16.** Utilisation d'un composé complexe de formule I selon la revendication 1 ou d'une composition selon au moins l'une des revendications 7 à 11 pour la préparation d'un médicament destiné à la prophylaxie et/ou la thérapie des inflammations ou des réactions allergiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0269926 A
- DE 19544905 A1
- DE 19922287 A1
- EP 03015616 A
- EP 796624 A
- JP 059137499 B
- JP 7075536 A
- JP 5186344 A
- DE 10244282
- DE 10232595
- US 6242099 B1
- WO 0009652 A
- WO 0072806 A
- WO 0071084 A
- EP 0671161 A
- DE 4116123 A
- DE OS4308282 A


### In der Beschreibung aufgeführte Nicht-Patentliteratur


- **K. Miyake ; H. Arima et al.** *Pharm. Dev. Techn.,* 2000, vol. 5 (3), 399-407
- **T. K. Nguyen ; H. Galons ; C. Chemtob.** *Congr. Int. Technol. Pharm.,* 1992, vol. 5, 408-16408416
- **S. Dimova ; R. Mugabowindekwe et al.** *Int. J. Pharm. 26381-2,* 2003, 95-103
- **R. Ficarra ; S. Tommasini et al.** *J. Pharm. Biomed. Analysis,* 2002, vol. 29 (6), 1005-1014
- **R.-L. Wang ; Yu Yang et al.** *Yingyong Huaxue,* 2002, vol. 19 (7), 702-704
- **K. Hostettmann ; M. Lederer ; A. Marston.** *Phytochemical Analysis,* 2000, 380-382
- **B. Vermes ; H. Wagner.** *Stud. Org. Chem. (Amsterdam),* 1981, vol. 11, 161-167
- **B. Vermes ; V.M. Chari ; H. Wagner.** *Helv. Chim. Acta,* 1981, vol. 64 (4), 1964-1967
- **H. Wagner ; H. Danninger ; O. Seligmann ; M. Nógrádi ; L. Farkas ; N. Farnsworth.** *Chem. Ber.,* 1978, vol. 103, 3768
- **D.Y. Gagniere ; P.J.A. Wottero.** *Carbohydrate Res.,* 1973, vol. 28, 1965
- **G. Zemplén.** *Chem. Ber.,* 1926, vol. 59, 1258
- **A.B. Foster et al.** *J. Chem. Soc.,* 1954, 3625-3629
- **P. Beraud et al.** *Tetrahedron Let.,* 1989, vol. 30 (3), 325-326
- **M. Goebel et al.** *Tetrahedron,* 1997, vol. 53 (9), 3123-3134
- **K. Lemanska ; H. Szymusiak ; B. Tyrakowska ; R. Zielinski ; I.M.C.M. Rietjens.** *Current Topics in Biophysics,* 2000, vol. 24 (2), 101-108
- **C.A. Rice-Evans ; N.J. Miller ; G. Paganga.** *Trends in Plant Science,* 1997, vol. 2 (4), 152-159
- **K. Lemanska ; H. Szymusiak ; B. Tyrakowska ; R. Zielinski ; A.E.M.F. Soffers ; I.M.C.M. Rietjens.** *Free Radical Biology&Medicine,* 2001, vol. 31 (7), 869-881
- **E. A. Galinski et al.** *Eur. J. Biochem.,* vol. 149 (9985), 135-139
- Colour Index Nummern (CIN) sind dem Rowe Colour Index. Society of Dyers and Colourists, 1971